(19) Europäisches Patentamt
European Patent Office
Office européen des brevets

(11) Veröffentlichungsnummer: **0 272 589 B1**

(12) # EUROPÄISCHE PATENTSCHRIFT

(45) Veröffentlichungstag der Patentschrift: **28.10.92**

(21) Anmeldenummer: **87118606.0**

(22) Anmeldetag: **15.12.87**

Die Akte enthält technische Angaben, die nach dem Eingang der Anmeldung eingereicht wurden und die nicht in dieser Patentschrift enthalten sind.

(51) Int. Cl.5: **C07D 487/22**, C07D 519/00, C08G 63/68, C08K 5/34, C07D 211/36, C07D 498/10, C07D 471/10, //(C07D487/22, 251:00,251:00,235:00,235:00), (C07D519/00,487:00,487:00), (C07D498/10,263:00,221:00), (C07D471/10,235:00,221:00)

(54) **Polyalkylpiperidinderivate mit Alkylenbrücken und ihre Verwendung als Stabilisator.**

(30) Priorität: **22.12.86 DE 3643890**

(43) Veröffentlichungstag der Anmeldung: **29.06.88 Patentblatt 88/26**

(45) Bekanntmachung des Hinweises auf die Patenterteilung: **28.10.92 Patentblatt 92/44**

(84) Benannte Vertragsstaaten: **BE CH DE FR GB IT LI NL**

(56) Entgegenhaltungen:
**EP-A- 0 019 578**
**EP-A- 0 213 570**
**DE-A- 2 326 010**
**DE-A- 2 930 397**
**DE-A- 3 523 679**
**FR-A- 2 291 203**
**EP-A- 0 079 850**
**DE-A- 2 233 121**
**DE-A- 2 924 894**
**DE-A- 3 208 570**
**DE-B- 2 227 689**
**GB-A- 2 157 294**

(73) Patentinhaber: **BASF Aktiengesellschaft**
**Carl-Bosch-Strasse 38**
**W-6700 Ludwigshafen(DE)**

(72) Erfinder: **Aumueller, Alexander, Dr.**
**An der Marlach 5**
**W-6705 Deidesheim(DE)**
Erfinder: **Neumann, Peter, Dr.**
**Franz Schubert-Str. 1**
**W-6908 Wiesloch(DE)**
Erfinder: **Trauth, Hubert**
**Milanstr. 5**
**W-6724 Dudenhofen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 272 589 B1

## Beschreibung

Es ist bekannt, daß 2,2,6,6-Tetraalkyl-piperidinderivate Lichtschutzmittel für organische Polymere sind. Unbefriedigend ist oft die Verträglichkeit mit Polyolefinen, die Dauer der Schutzwirkung, die Neigung zur Flüchtigkeit und die Eigenfarbe der Substanzen.

In der FR-A-1 291 203 sind Glycolurilderivate der Formel

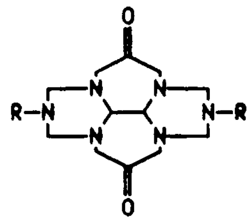

beschrieben, worin R einen gegebenenfalls substituierten Kohlenwasserstoffrest mit bis zu 22 Kohlenstoffatomen bedeutet. Diese Verbindungen werden als oberflächenaktive Mittel für die Textilindustrie und als Korrosionsinhibitoren vorgeschlagen.

Der Erfindung liegt die Aufgabe zugrunde, neue Polyalkylpiperidinderivate zur Verfügung zu stellen, welche die vorstehenden Nachteile vermeiden.

Diese Aufgabe wird gelöst mit Polyalkylpiperidinderivaten der allgemeinen Formel I

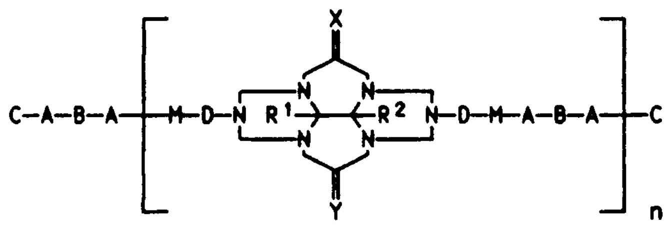

(I)

worin

| | |
|---|---|
| n | für eine ganze Zahl von 1 bis 70 steht, |
| $R^1$ und $R^2$ | unabhängig voneinander Wasserstoff, $C_1$-$C_6$-Alkyl, $C_7$-$C_{12}$-Aralkyl, Aryl oder Carbonester bedeuten oder zusammen eine Tetra-, Penta- oder Hexamethylengruppierung darstellen, oder gemeinsam für einen gegebenenfalls durch $C_1$-$C_6$-Alkyl, $C_1$-$C_6$-Alkoxy, $C_2$-$C_{12}$-Carbonester, $C_1$-$C_{12}$-C-Acyl, Halogen, Nitro, Carboxyl, Amino, Hydroxy, -SH, -S-$C_1$-$C_4$-Alkyl, Phenyl, $C_2$-$C_6$-Alkenyl, (Poly)ethoxy, (Poly)amino substituierten Rest der Formel 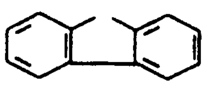 stehen, |
| X und Y | unabhängig voneinander Sauerstoff, Schwefel oder $NR^7$ darstellen, wobei $R^7$ für Wasserstoff, $C_1$-$C_8$-Alkyl, oder $C_7$-$C_{12}$-Aralkyl steht, |
| M | für |

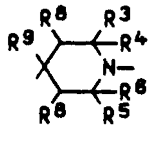

steht, worin $R^3$, $R^4$, $R^5$ und $R^6$ gleich oder verschieden sind und jeweils Alkyl bedeuten, oder worin $R^3$ und $R^4$ sowie $R^5$ und $R^6$ zusammen mit dem Kohlenstoffatom, an das sie gebunden sind, einen 5- oder 6-gliedrigen Ring bilden, und worin $R^8$ für Wasserstoff oder Alkyl steht, oder mit dem zugehörigen Kohlenstoffatom eine
$>C=O$ Gruppe
bildet, und worin $R^9$ Wasserstoff darstellt oder eine weitere Bindung zu einem spiroverknüpften Brückenglied -B- (-A- bedeutet in diesem Fall eine direkte Bindung) bedeutet,

D jeweils gleich oder verschieden ist und eine Gruppe-$(-CH_2-)_m$ darstellt, worin m eine Zahl von 1 bis 20 bedeutet, oder, falls der Rest M über das Stickstoffatom mit -A- verbunden ist, auch eine direkte Bindung sein kann,
wobei mindestens eine Gruppierung -M-D- im Molekül vorliegt und in dieser der Rest -M- über sein Stickstoffatom mit -D- verbunden ist,

A jeweils gleich oder verschieden ist und für Sauerstoff, gegebenenfalls einfach durch $C_1$-$C_6$-Alkyl, $C_1$-$C_{12}$-C-Acyl oder Acylgruppen der Formel $CO-(CH_2)_m-H$, wobei m eine Zahl von 0 bis 20 ist, substituierten Stickstoff, Carboxyl, Carbonyl, Sulfonyl, Sulfonamido, eine Gruppe der Formeln

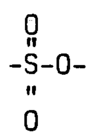 , 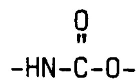 oder 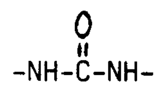

oder eine direkte Bindung steht,

B ein Brückenglied der Formeln

$-(CH_2)_p-$, $-(CH_2)_pCH=CH-$, $-(CH_2)_pC\equiv C-$, 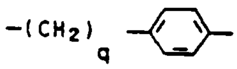, 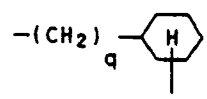,

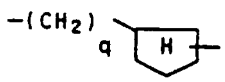, 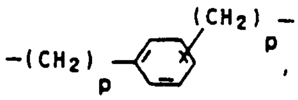, $-(CH_2)_2O$ , $-(CH_2)_2O(CH_2)_2-$,

$-(CH_2)_3O(CH_2)_2-$, 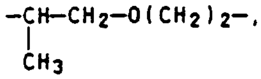, 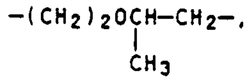, 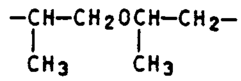,

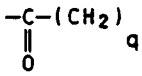, 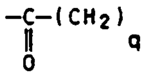, 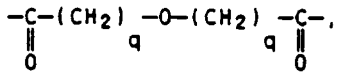,

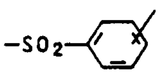, 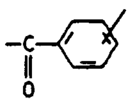, 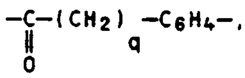, 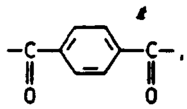, 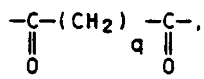,

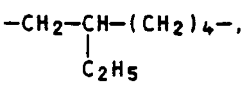, 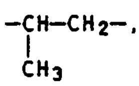, $-CH_2-CH=CH-$, 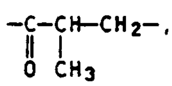, 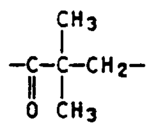,

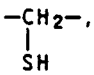, 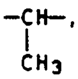, 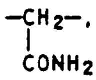, 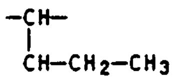, 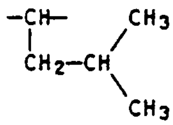, 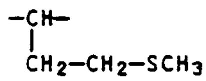,

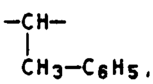, 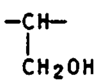, 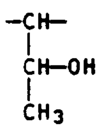, 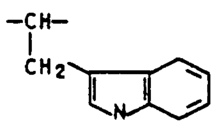, 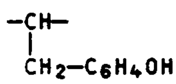, 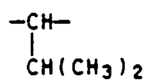,

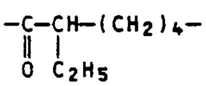, 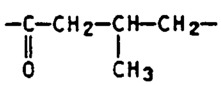, 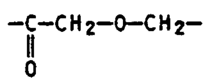 oder 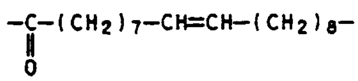,

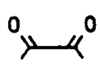, 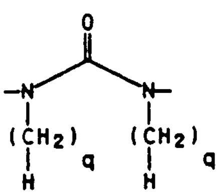, 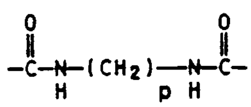,

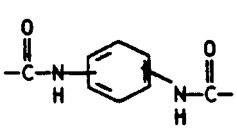, 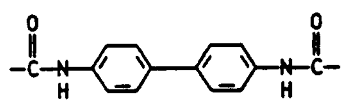,

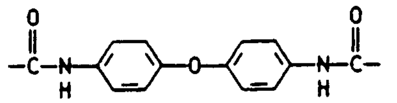 , 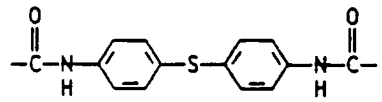 ,

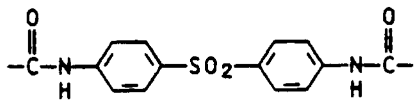 , 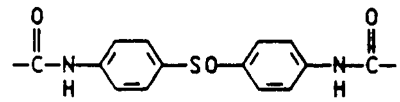 ,

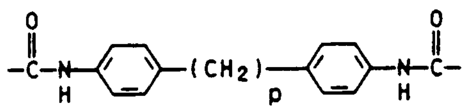 , 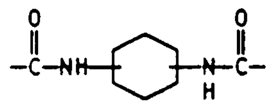 ,

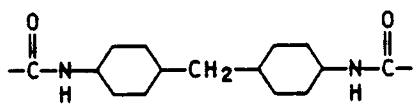 , 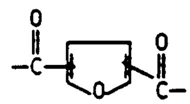 , 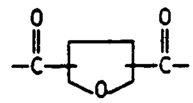 ,

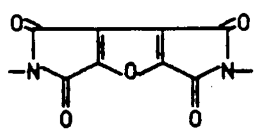 , 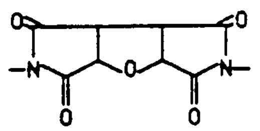 , 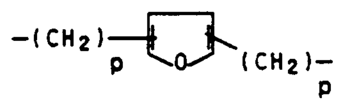 ,

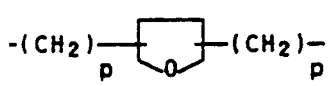 , 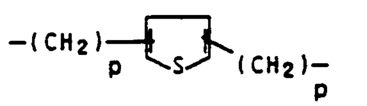 , 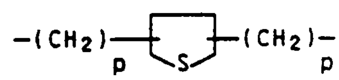 ,

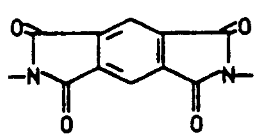 , 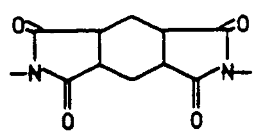 ,

wobei p = 1 bis 20 und q = 0 bis 20 ist,
und wobei das Brückenglied B mit dem Rest -M- auch durch eine Spiroverknüpfung der Struktur

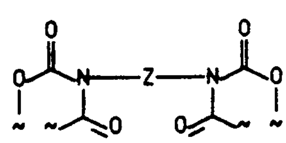

(Spiroverknüpfung an -M-) (Spiroverknüpfung an -M-) verbunden sein kann,
worin Z ein Brückenglied darstellt und für $-(CH_2)_k-$ (k = 0 bis 20),

**Phenylen,** 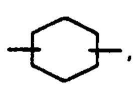, **Xylylen,** 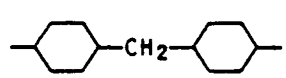 **oder** 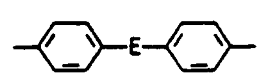

(E = O, N, S, $SO_2$, SO, $-CH_2-$, $-CH_2-CH_2-$, direkte Bindung) steht, oder eine direkte Bindung bedeutet, und

C für Wasserstoff, $C_1$-$C_{20}$-C-Acyl, Chlor, Brom, Iod, Hydroxy, Amino, substituiertes Amino der Formel $H-(CH_2)_r-NH-$ (r = 0 bis 20), Alkoxy der Formel $H-(CH_2)_r-O-$ (r = 0 bis 20),

Carboxyl, Carbonester, Cyan, Sulfonamido, Carbonyl-Gruppierungen der Formel

$H-(CH_2)_r-CO-$, 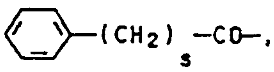, 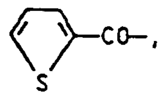,

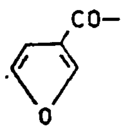, 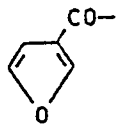, 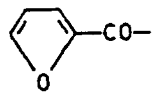, wobei r = 0 bis 20 und s = 1 bis 5 ist,

eine Harnstoffgruppierung der Formel

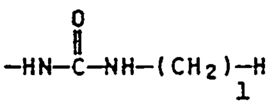, 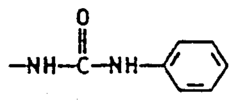, 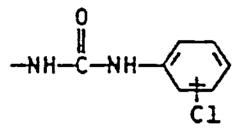,

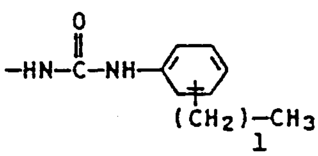, 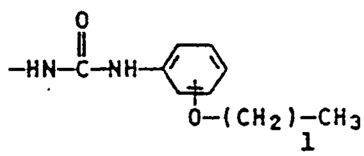,

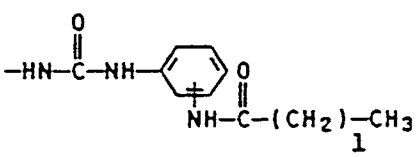, 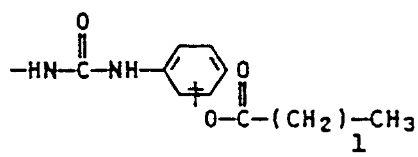,

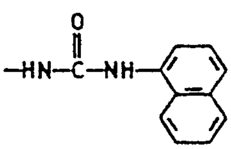, 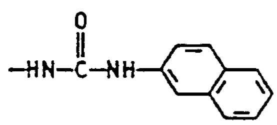,

wobei l = 0 bis 22 ist,
oder eine Urethangruppe der Formel

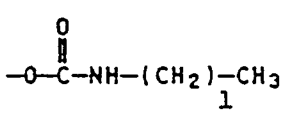, 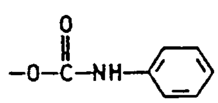, 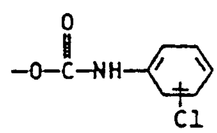,

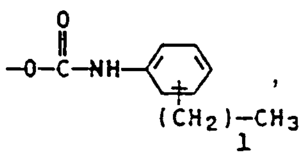, 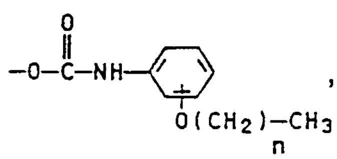, 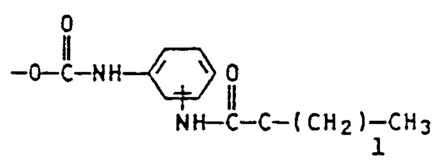

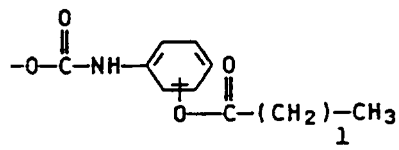 , 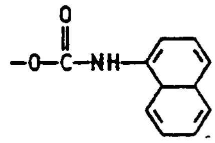 ,

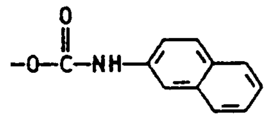 ($l$ = 0 bis 22),

oder eine Aryl- oder Heteroarylcarbonylgruppierung der Formel

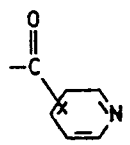 , 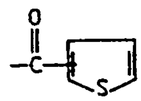 , 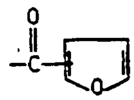 , 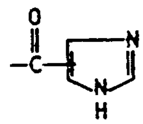 , 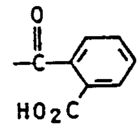 ,

steht, oder eine Gruppierung der Formel

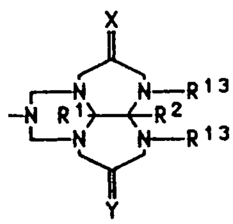

darstellt, worin die Reste $R^{13}$ gleich oder verschieden sind und für Wasserstoff oder $CH_2OR^{14}$ stehen, worin $R^{14}$ Wasserstoff oder einen $C_1$-$C_{22}$-Alkylrest, der durch Ethersauerstoff, Stickstoff oder Schwefel unterbrochen sein kann, bedeutet,
oder worin die Reste $R^{13}$ gemeinsam für eine Gruppierung

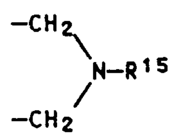

stehen können, worin $R^{15}$ die Bedeutungen 1)$C_1$-$C_{22}$-Alkyl, das durch Ethersauerstoff, Schwefel oder Stickstoff unterbrochen sein kann, 2)$C_1$-$C_{22}$-Alkenyl, 3)$C_3$-$C_{12}$-Cycloalkyl, 4)gegebenenfalls substituiertes $C_7$-$C_{12}$-Aralkyl, 5)$C_3$-$C_{12}$-Alkinyl, 6)nichtaromatischer Heterocyclus, 7)$C_1$-$C_{22}$-Alkyl oder Aralkyl, die einen Heterocyclus enthalten oder 8)$C_1$-$C_{22}$-Alkyl, das Hydroxy, Thiol, Cyan, Carboxyl, Carbonester, Carbamoyl, welches durch $C_1$-$C_6$-Alkyl, $C_1$-$C_6$-Alkoxy, $C_2$-$C_{12}$-Carbonester, $C_1$-$C_{12}$-C-Acyl, Halogen, Nitro, Carboxyl, Amino, Hydroxy, -SH, -S-$C_1$-$C_4$-Alkyl, Phenyl, $C_2$-$C_6$-Alkenyl, (Poly)ethoxy oder (Poly)amino substituiert sein kann, Brom, Chlor, Iod, Sulfon, Sulfonoxid, Sulfonyl, Sulfonamid, welches durch $C_1$-$C_6$-Alkyl, $C_1$-$C_6$-Alkoxy, $C_2$-$C_{12}$-Carbonester, $C_1$-$C_{12}$-C-Acyl, Halogen, Nitro, Carboxyl, Amino, Hydroxy, -SH, -S-$C_1$-$C_4$-Alkyl, Phenyl, $C_2$-$C_6$-Alkenyl, (Poly)ethoxy oder (Poly)amino substituiert sein kann, einen obengenannten Urethanrest oder Harnstoffrest enthält, besitzt,
oder worin in der allgemeinen Formel (I) die Gruppierung -M-A-B-A-C für eine Gruppe der allgemeinen Formeln

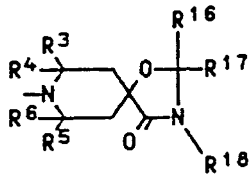
,
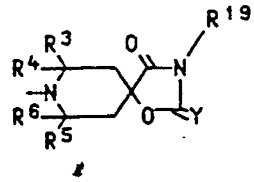

oder

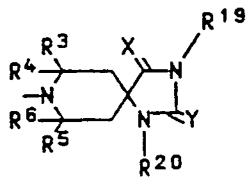

steht,
worin

| | |
|---|---|
| $R^3$ bis $R^6$ | sowie X und Y die oben angegebenen Bedeutungen besitzen, |
| $R^{16}$ und $R^{17}$ | unabhängig voneinander Wasserstoff, gegebenenfalls durch Ethersauerstoff, Schwefel oder Stickstoff unterbrochenes oder durch Cyan-, Carboxyl-, Carbamoyl-, Carbonester- oder Ketogruppen substituiertes $C_1$-$C_{22}$-Alkyl, $C_3$-$C_{12}$-Cycloalkyl, Bicycloalkyl, Tricycloalkyl, gegebenenfalls durch Chlor, $C_1$-$C_{22}$-Alkyl oder $C_1$-$C_{22}$-Alkoxy substituiertes Aryl, oder $C_7$-$C_{22}$-Aralkyl darstellen, oder worin |
| $R^{16}$ und $R^{17}$ | zusammen mit dem sie bindenden Kohlenstoffatom eine $C_5$-$C_{18}$-Cycloalkylgruppe, die durch bis zu 4 $C_1$-$C_4$-Alkylgruppen substituiert sein kann oder eine Gruppe der Formel |

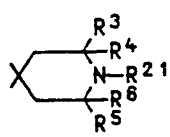

| | |
|---|---|
| | bilden, worin |
| $R^{21}$ | Wasserstoff, $C_1$-$C_{22}$-Alkyl, das durch Ethersauerstoff, Schwefel oder Stickstoff unterbrochen oder durch Cyan, Hydroxyl, Thiol, Carboxyl-, Carbamoyl- oder Carbonestergruppen substituiert sein kann, $C_1$-$C_{22}$-Alkenyl oder $C_1$-$C_{22}$-C-Acyl bedeutet, und $R^3$, $R^4$, $R^5$, $R^6$ die vorstehenden Bedeutungen besitzen, |
| $R^{18}$ | Wasserstoff, $C_1$-$C_{22}$-Alkyl, das durch Ethersauerstoff, Schwefel oder Stickstoff unterbrochen oder durch Cyan, Carboxyl-, Carbamoyl, Carbonester, Hydroxy-, Thiol- oder Aminogruppen substituiert sein kann oder $C_1$-$C_{22}$-C-Acyl, |
| $R^{19}$ | Wasserstoff, $C_1$-$C_{22}$-Alkyl, das durch Ethersauerstoff, Schwefel oder Stickstoff unterbrochen oder durch Cyan- oder Carbonestergruppen substituiert sein kann, Aryl, das gegebenenfalls durch Chlor, $C_1$-$C_{22}$-Alkyl oder $C_1$-$C_{22}$-Alkoxy substituiert sein kann, $C_7$-$C_{22}$-Aralkyl oder $C_3$-$C_{12}$-Cycloalkyl und |
| $R^{20}$ | Wasserstoff oder gegebenenfalls verzweigtes Alkyl bedeuten, |

sowie die Säureadditionssalze und Hydrate dieser Verbindungen.

Die Erfindung betrifft auch Gemische der erfindungsgemäßen Verbindungen der allgemeinen Formel I.

Bevorzugt sind Verbindungen mit n = 1 bis 20 und insbesondere mit n = 1.

Einzelne Reste $R^1$ und $R^2$ sind neben Wasserstoff z.B.: Methyl, Ethyl, Propyl, Butyl, Pentyl, Hexyl, Benzyl, Phenylethyl, Phenylpropyl, Phenylbutyl, Methylbenzyl, Phenyl, Tolyl, Carbomethoxy, Carboethoxy, Carbopropoxy oder Carbobutoxy.

Bevorzugt sind für $R^1$ und $R^2$ Methyl, Ethyl, Benzyl, Phenyl und insbesondere Wasserstoff, Methyl und Phenyl.

Der Begriff "Alkyl" steht für geradkettige oder verzweigte Alkylreste, insbesondere $C_1$-$C_{22}$-Alkyl,

bevorzugt $C_1$-$C_6$-Alkyl, besonders bevorzugt $C_1$-$C_4$-Alkyl. So sind beispielsweise Alkylreste für $R^3$ bis $R^6$ $C_1$- bis $C_4$-Alkyl, also Methyl, Ethyl, Propyl oder Butyl. Im Falle der Reste $R^3$ bis $R^6$ können zwei benachbarte Reste auch eine Tetra-, Penta- oder Hexamethylengruppierung bilden.

Vorzugsweise sind $R^3$ bis $R^6$ Methyl.

X und Y sind unabhängig voneinander insbesondere Sauerstoff, daneben auch Schwefel oder $NR^7$, wobei $R^7$ die vorstehenden Bedeutungen besitzt.

Besonders bevorzugt für A ist Sauerstoff.

Brückenglieder B sind zweiwertige aliphatische, araliphatische oder aromatische Gruppen, die auch Heteroatome, nämlich Sauerstoff, Stickstoff oder Schwefel, enthalten können, sowie insbesondere eine direkte Bindung.

Insbesondere sind Alkylen-, Cycloalkylen-, Aralkylen-, durch CO oder $SO_2$ unterbrochene oder substituierte Alkylen-, Aralkylen- oder Arylenreste zu nennen.

Bevorzugt steht C für $CH_3CO$, $CH_3CH_2CO$ und insbesondere Wasserstoff.

Soweit in den erfindungsgemäßen Verbindungen Cycloalkylreste oder Bicycloalkylreste vorliegen, können diese beispielsweise die folgenden sein:

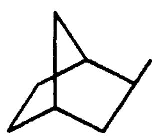 , 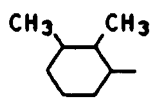 , 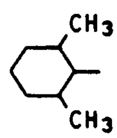 , 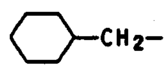 ,

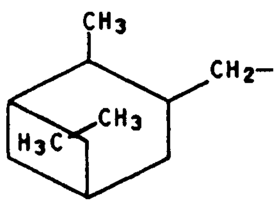 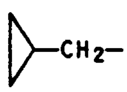 , 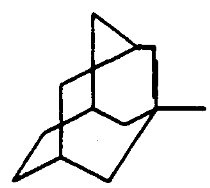

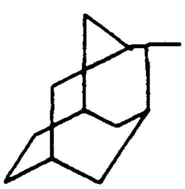 , 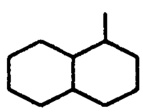 , 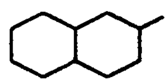 , 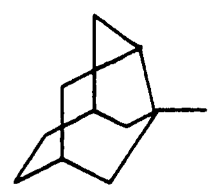

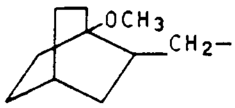 , 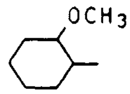 , 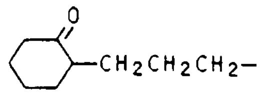 .

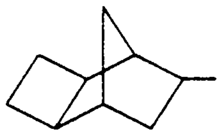 , 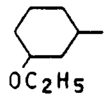 , 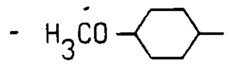 ,

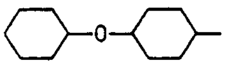 , 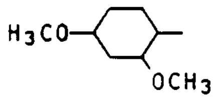 , 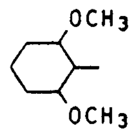 ,

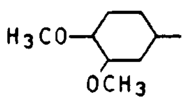 , 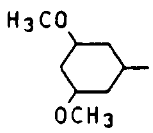 , 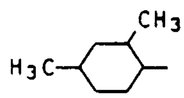 , 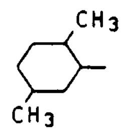

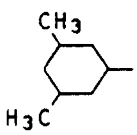 , 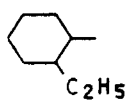 , 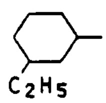 , 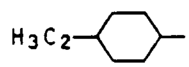 , 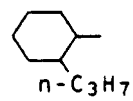 ,

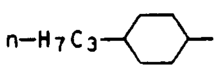 , 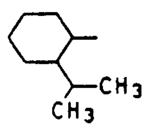 , 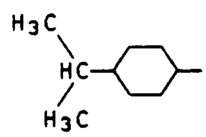 , 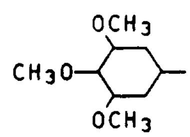 .

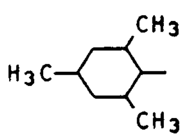 , 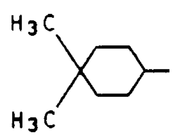 , 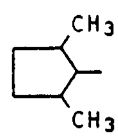 ,

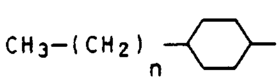 , 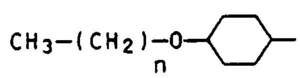 .

Soweit in den erfindungsgemäßen Verbindungen Hydroxy-substituierte oder Carboxyl-substituierte Alkylreste vorliegen, können diese beispielsweise die folgenden sein:

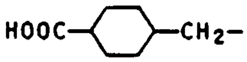 , $HOOC-(CH_2)_n-$ (n = 1 bis 20) , 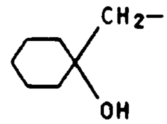 , 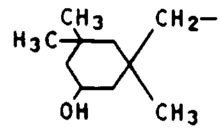 ,

$HO-CH_2CH_2O-CH_2CH_2-$ , $HO-(CH_2)_n-$ (n = 1 bis 20), 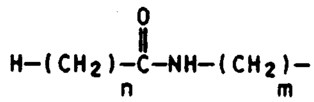 ,

und

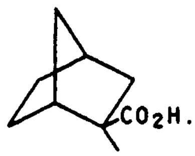

Soweit in den erfindungsgemäßen Verbindungen Aralkylreste vorliegen, können diese beispielsweise die folgenden sein:

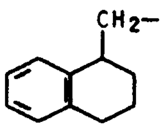 , 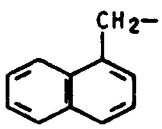 , 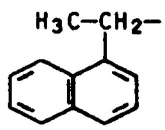 , 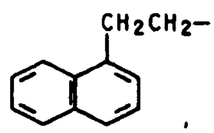 ,

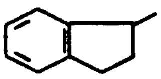 , 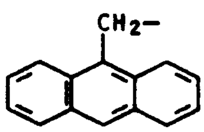 , 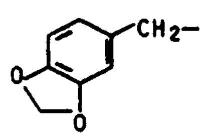 ,

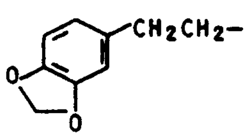 , 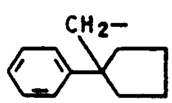 ,

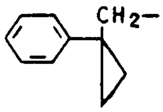 , 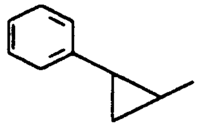 , 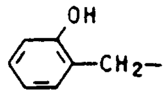 ,

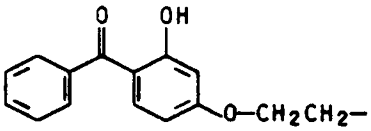 , 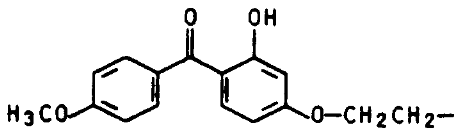 ,

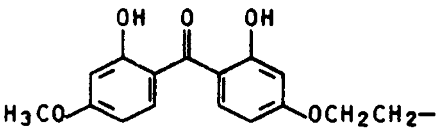 , 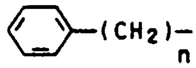 (n = 1 bis 20).

Soweit in den erfindungsgemäßen Verbindungen nicht aromatische Heterocyclen vorliegen, können diese beispielsweise die folgenden sein:

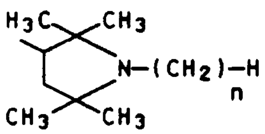 , 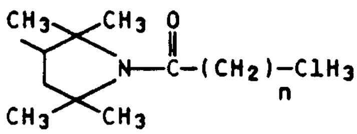 , 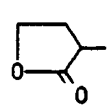 ,

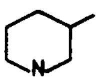 , 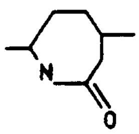 , 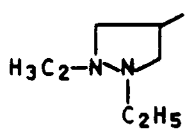 , 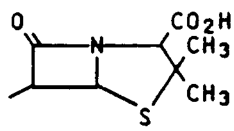

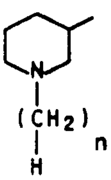 , 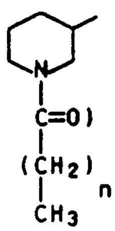 , 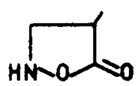 , 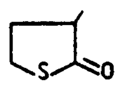

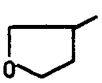 , 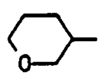 , 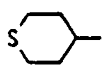 , 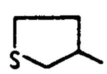 ,

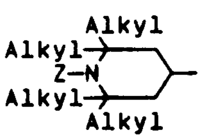 (Z = Wasserstoff, Alkyl, Acyl, HO—) .

Der Begriff "-C-Acyl" steht beispielsweise für $C_1$-$C_{20}$-C-Acyl, insbesondere für Reste der Formel

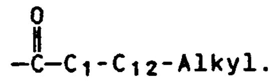

Verbindungen der allgemeinen Formel (I) mit n = 1 können durch Reaktion von Verbindungen der allgemeinen Formel (I') mit Verbindungen der allgemeinen Formel (II) analog dem Verfahren der FR-A-22 91 203 hergestellt werden.

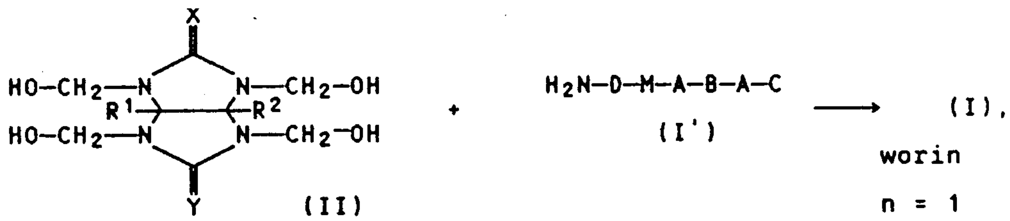


(II) + (I') → (I),

Verbindungen der allgemeinen Formel I mit n = 1 können durch Reaktion von Verbindungen der allgemeinen Formel (I') mit D ungleich direkte Bindung und Verbindungen der allgemeinen Formel (I') mit D gleich direkte Bindung hergestellt werden. Dabei können die Verbindungen der allgemeinen Formel (I') gleichzeitig oder in beliebiger Reihenfolge zu Verbindungen der allgemeinen Formel (I) gegeben werden. In diesem Falle wird man in der Regel ein Gemisch aus den denkbaren Reaktionsprodukten erhalten. Gewünschtenfalls können diese Gemische in an sich bekannter Weise in die Einzelverbindungen aufgetrennt werden.

Die Verbindungen der allgemeinen Formel (I') können dabei auch in situ durch Reaktion von Verbindungen der allgemeinen Formel (I''') mit Formaldehyd oder einer Formaldehydquelle erzeugt werden.

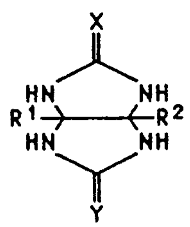 (I'''),

Verbindungen der allgemeinen Formel (I) mit freien Amino- oder Hydroxygruppen können durch literaturbekannte Verfahren in die entsprechenden acylierten und alkylierten Produkte überführt werden.

Verwendet man als Acylierungs- bzw. Alkylierungsmittel Verbindungen mit mehr als einer reaktiven Gruppe, so erhält man Verbindungen der allgemeinen Formel (I) mit n größer als 1.

Entsprechendes gilt für Reaktionen mit Isocyanaten zu den entsprechenden Urethanen und Harnstoffen. Verbindungen der allgemeinen Formel (I) mit freien Carboxylgruppen oder Carbonestergruppen aus niederen Alkoholen können durch Veresterung, Aminolyse oder Umesterung in die entsprechenden Ester und Amide umgewandelt werden. Verwendet man bei der Reaktion als Alkohol, Amin oder Aminoalkohol eine Verbindung mit mehr als einer reaktiven Gruppe, so erhält man Verbindungen der allgemeinen Formel (I) mit n größer als 1.

Verbindungen der allgemeinen Formel (I) mit n größer als 1 können auch durch Reaktion von Verbindungen der allgemeinen Formel (II) mit Verbindungen der allgemeinen Formel (I'') hergestellt werden.

$H_2N$-D-M-A-B-A-M-D-$NH_2$ (I'')

Durch anschließende Zugabe eines Amins $R^{15}$-$NH_2$ kann dabei ein Endgruppenverschluß erfolgen. Auch hier können die Verbindungen der allgemeinen Formel (II) in situ durch Reaktion von Verbindungen der allgemeinen Formel (I''') mit Formaldehyd oder einer Formaldehydquelle erzeugt werden.

Die erfindungsgemäßen Verbindungen können in Form der freien Basen oder als Salze vorliegen. Geeignete Anionen stammen z. B. von anorganischen Säuren und insbesondere organischen Carbonsäuren sowie organischen Sulfonsäuren.

Die erfindungsgemäßen Verbindungen besitzen außerordentlich gute stabilisierende Eigenschaften, haben keine Eigenfarbe, sind gut verträglich mit organischen Polymeren und zeigen einen niedrigen Dampfdruck.

Anorganische Anionen sind z. B.: Chlorid, Bromid, Sulfat, Methosulfat, Tetrafluoroborat, Phosphat oder Rhodanid.

Carbonsäure-Anionen sind beispielsweise: Formiat, Acetat, Propionat, Hexanonat, Cyclohexanonat, Lactat, Stearat, Dodecylbenzoat, Benzoat, Acrylat, Methacrylat, Zitrat, Malonat oder Succinat sowie Anionen von Polycarbonsäuren mit bis zu 3000 COOH-Gruppen.

Sulfonsäure-Anionen sind beispielsweise Benzolsulfonat oder Tosylat.

Die erfindungsgemäßen Verbindungen eignen sich zum Stabilisieren von organischem Material, speziell von Kunststoffen, gegen den Abbau durch Licht und Wärme. Sie sind auch wirksam als Metalldesaktivator. Sie werden den zu stabilisierenden Kunststoffen in einer Konzentration von 0,01 bis 5 Gew.-%, vorzugsweise 0,02 bis 1 Gew.-% vor, während oder nach der Polymerbildung zugesetzt.

Zur Vermischung der erfindungsgemäßen Verbindungen mit den zu stabilisierenden Kunststoffen können alle bekannten Vorrichtungen und Methoden zum Einmischen von Stabilisierungsmitteln oder anderen Zusätzen in Polymere angewandt werden.

Die durch eine der erfindungsgemäßen Verbindungen stabilisierten Kunststoffe können gegebenenfalls noch weitere Additive enthalten, beispielsweise Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die den Kunststoffen neben den erfindungsgemäßen Verbindungen zugesetzt werden können, sind z. B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-β-(3,5-di-tert.-butly-4-hydro-xyphenyl)-propionat, 1,1,3-Tris-(2-methyl-4-hydroxy-5-tert.-butyl-phenyl)-butan, 1,3,5-Trimethyl-2,4,6-tris-(3,5-di-tert.-butyl-4-hydroxy-benzyl)-benzol, 1,3,5-Tris-(3,5-di-tert.-butyl-4-hydroxybenzyl)-isocyanurat, 1,3,5-Tris-[β-(3,5-di-tert.-butyl-4-hydroxyphenyl)-propionyloxy-ethyl]-iso cyanurat, 1,3,5-Tris-(2,6,di-methyl-3-hydroxy-4-tert.-butylbenzyl)-isocyanurat und Pentaerythrit-tetrakis-[β-(3,5-di-tert.-butyl-4-hydroxyphenyl)-propionat] erwähnt.

Als phosphorhaltige Antioxidantien seien beispielsweise Tris-(nonyl-phenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4'-biphenylendiphosphit erwähnt.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat) und Pentaerythrittetrakis-(β-hexylthiopropionat) erwähnt.

Weitere Antioxidantien und Lichtstabilisatoren, die zusammen mit den erfindungsgemäßen Verbindungen verwendet werden können, sind z. B. 2-(2'-Hydroxyphenyl)-benztriazole, 2-Hydroxybenzophenone, Arylester von Hydroxybenzoesäuren, α-Cyanozimtsäurederivate, Nickelverbindungen oder Oxalsäuredianilide.

Als organische Polymere, die durch die erfindungsgemäßen Verbindungen stabilisiert werden können, seine beispielsweise genannt:

Polymere von Mono- und Diolefinen, wie z. B. Polyethylen niedriger oder hoher Dichte, lineares Polyethylen niedriger Dichte, Polypropylen, Polyisobutylen, Polybuten-1, Polyisopren, Polybutadien sowie Copolymerisate von Mono- oder Diolefinen oder Mischungen der genannten Polymeren;

Copolymerisate von Mono- oder Diolefinen mit anderen Vinylmonomeren wie z. B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polystyrol;

Copolymere von Styrol oder α-Methylstyrol mit Dienen oder Acrylderivaten, wie z. B. Styrol-Butadien, Styrol-Acrylnitril, Styrol-Ethylmethacrylat. Styrol-Butadien-Ethylacrylat. Styrol-Acrylnitril-Methacrylat;

ABS-, MBS- oder ähnliche Polymere;

Halogenhaltige Polymere, wie z. B. Polyvinylchlorid, Polyvinylfluorid, Polyvinylidenfluorid sowie dere Copolymere;

Polymere die sich von α,β-ungesättigten Säuren und deren Derivaten ableiten, wie Polyacrylate und Polymethacrylate, Polyacrylamide und Polyacrylnitrile;

Polymere, die sich von ungesättigten Alkoholen und Aminen bzw. deren Acrylderivaten oder Acetalen ableiten, wie Polyvinylalkohol oder Polyvinylacetat;

Polyurethane, Polyamide, Polyharnstoffe, Polyester, Polycarbonate, Polysulfone, Polyethersulfone und Polyetherketone.
Weitere organische Polymere, die mit den erfindungsgemäßen Verbindungen stabilisiert werden können, stellen Industrielackierungen dar. Unter diesen sind Einbrennlackierungen, unter diesen wiederum Fahrzeuglackierungen, vorzugsweise Zweischichtlackierungen, besonders hervorzuheben.

Auch hier können zusätzlich die bereits aufgeführten Antioxidantien und Lichtschutzmittel verwendet werden.

Die erfindungsgemäßen Verbindungen können in fester oder gelöster Form dem Lack zugesetzt werden. Ihre gute Löslichkeit in Lacksystemen ist dabei von besonderem Vorteil.

Bevorzugt ist die Verwendung der erfindungsgemäßen Verbindungen in Polyolefinen, vorzugsweise Ethylen- und Propylenpolymerisaten.

Weiterhin ist bevorzugt die Verwendung der erfindungsgemäßen Verbindungen in Polyurethanen. Vorzugsweise werden die erfindungsgemäßen Verbindungen in Kombination mit UV-Absorbern oder Antioxidantien oder UV-Absorbern und Antioxidantien eingesetzt.

Die Erfindung wird durch die nachstehenden Beispiele weiter erläutert.

Beispiel 1

34 g (0,17 Mol) N-$\beta$-Aminoethyl-4-hydroxy-2,2,6,6-tetramethylpiperidin und 44,5 g (0,085 Mol) einer 50 %igen wäßrigen Lösung von Tetramethylolacetylendiharnstoff werden in 75 ml Wasser 5,5 h auf 80 °C erwärmt. Man saugt ab, trocknet und kristallisiert aus Acetonitril um. Die Verbindung kristallisiert mit 1 Mol Wasser. Man erhält 23,9 g (46 % d. Th.) farblose Kristalle vom Schmp. 263 °C (Zers.).

| | | | | |
|---|---|---|---|---|
| Ber.: | C 59,2 | H 9,3 | N 18,4 | O 13,1 |
| Gef.: | C 58,7 | H 9,4 | N 18,1 | O 13,0 |

Die Verbindung kann durch Kochen in Toluol am Wasserabscheider von Wasser befreit werden.

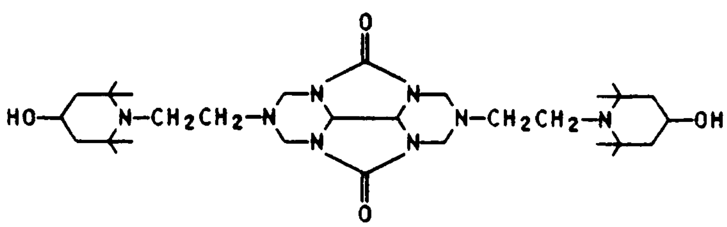

Beispiel 2

10,0 g (16,9 mmol) der Verbindung aus Beispiel 1 werden in 150 ml Dichlormethan gelöst und auf 0 bis 10 °C gekühlt. Bei dieser Temperatur werden 3,5 g (45,0 mmol) Acetylchlorid zugetropft. Nach Stehen über Nacht wird mit verdünnter Natronlauge auf pH 10 neutralisiert, die organische Phase eingeengt. Nach Umkristallisation aus Methylcyclohexan erhält man 10,0 g (88 %) Diacetylverbindung als farblose Kristalle vom Schmp. 236 -238 °C.

| | | | | |
|---|---|---|---|---|
| Ber.: | C 60,5 | H 8,7 | N 16,6 | O 14,2 |
| Gef.: | C 60,4 | H 8,9 | N 16,2 | O 14,5 |

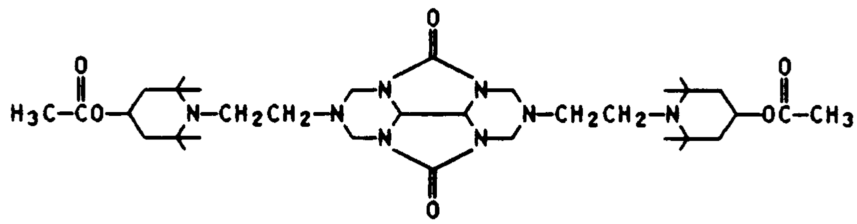

Beispiel 3

10,0 g der Verbindung aus Beispiel 1 werden mit 5,9 g Propionsäureanhydrid analog Beispiel 2 umgesetzt. Nach Neutralisieren wird mit n-Butanol extrahiert, die Butanolphase eingeengt und der Rückstand aus Methylcyclohexan umkristallisiert. Man erhält 3,5 g (30 %) Dipropionylverbindung als farblose Kristalle vom Schmp. 239 ° C.

| | | | | |
|---|---|---|---|---|
| Ber.: | C 61,5 | H 8,9 | N 16,0 | O 13,7 |
| Gef.: | C 61,6 | H 9,1 | N 16,2 | O 13,7 |

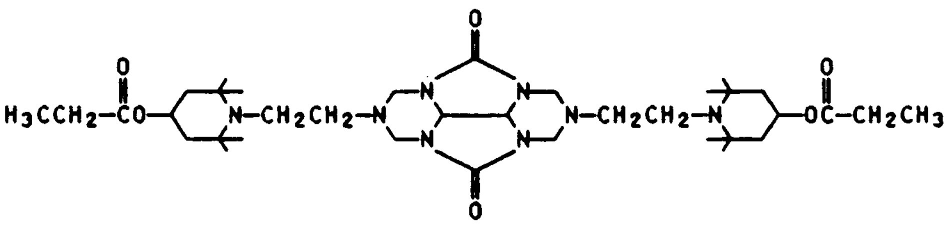

Beispiel 4

a) 84 g (= 0,23 Mol) N-[2,2,6,6-Tetramethyl-4-piperidinyl]-myristinsäureamid werden in 150 ml Ethanol gelöst. Man tropft 28 g einer 70 %igen wäßrigen Glykolsäurenitrillösung zu und erwärmt 40 h auf 70 ° C. Der ausgefallene Niederschlag wird abgesaugt, mit Ethanol gewaschen und 2 mal aus Ethanol umkristallisiert.

Man erhält 33 g N-[1-Cyanmethyl-2,2,6,6-tetramethyl-4-piperidinyl]-myristinsäureamid vom Schmp. 106 ° C.

| | | | | |
|---|---|---|---|---|
| Ber.: | C 74,0 | H 11,7 | N 10,4 | O 3,9 |
| Gef.: | C 74,2 | H 11,7 | N 10,4 | O 4,0 |

b) 25 g des Produkts aus a) werden in 300 ml Toluol zusammen mit 20 g Ammoniak und 5 g Raney-Nickel bei 250 bar hydriert. Man filtriert, engt ein und kristallisiert den Rückstand aus Acetonitril um. Man erhält 19 g N-[1-β-Aminoethyl-2,2,6,6-tetramethyl-4-piperidinyl]-myristinsäureamid vom Schmp. 88-89 ° C.

c) 9,5 g des Produktes aus b) und 6,0 g einer 50 %igen wäßrigen Tetramethylolacetylendiharnstofflösung werden in 70 ml Isobutanol 5 h am Wasserabscheider gekocht.

Man engt ein und kristallisiert den Rückstand aus Acetonitril um. Man isoliert 6,6 g der Verbindung der Formel

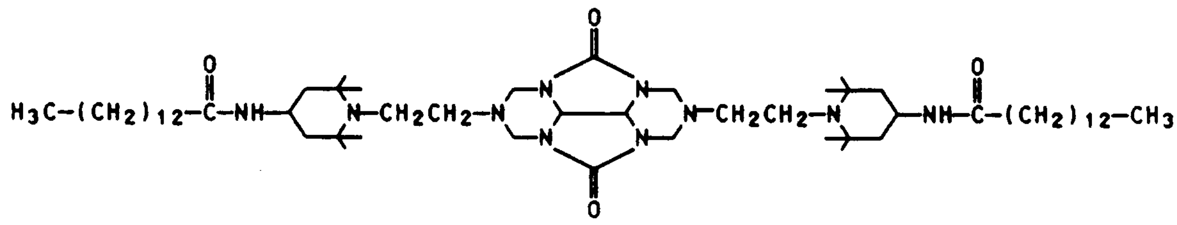

vom Schmp. 135-136 ° C.

| | | | | |
|---|---|---|---|---|
| Ber.: | C 69,0 | H 10,8 | N 13,9 | O 6,3 |
| Gef.: | C 69,0 | H 10,9 | N 13,0 | O 6,8 |

Beispiel 5

a) 96 g N-[2,2,6,6-Tetramethyl-4-piperidinyl]-pentadecansäureamid werden wie in Beispiel 4a umgesetzt. Nach Umkristallisation des Rohproduktes aus Ethanol erhält man 62 g N-[1-Cyanmethyl-2,2,6,6-tetramethyl-4-piperidinyl]pentadecansäureamid vom Schmp. 101 ° C.

| | | | | |
|---|---|---|---|---|
| Ber.: | C 74,5 | H 11,7 | N 10,0 | O 3,8 |
| Gef.: | C 74,5 | H 11,8 | N 9,9 | O 3,9 |

b) 40 g des Produktes aus a) werden wie in Beispiel 4b angegeben hydriert. Das Rohprodukt wird aus Acetonitril umkristallisiert, man erhält 35,8 g N-[1-β-Aminoethyl-2,2,6,6-tetramethyl-4-piperidinyl]-pentadecan-säureamid vom Schmp. 65 ° C.

c) 35 g des Produktes aus b) werden mit Tetramethylolacetylendiharnstoff analog Beispiel 4c zur Reaktion gebracht. Nach Umkristallisation aus Acetonitril erhält man 28 g der Verbindung der Formel

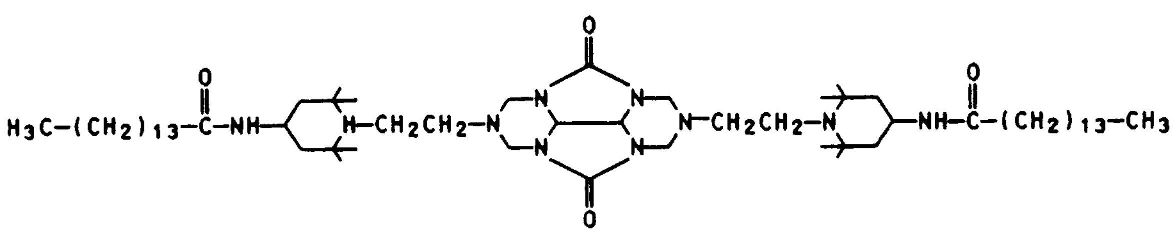

vom Schmp. 157-158 ° C.

| | | | | |
|---|---|---|---|---|
| Ber.: | C 69,4 | H 10,9 | N 13,5 | O 6,2 |
| Gef.: | C 69,0 | H 10,9 | N 13,1 | O 6,4 |

Beispiel 6

a)

15 ml mit Kaliumcarbonat gesättigtes Ethanol, 18 g Paraformaldehyd, 1,43 g Kaliumcarbonat und 51 g Acetoncyanhydrin wurden 2 h bei 25 ° C gerührt. Man stellte mit 85 %iger Phosphorsäure auf pH = 6, gab 75 ml Ethanol und 88 g N-[2,2,6,6-Tetramethyl-4-piperidinyl]-pelargonsäureamid zu und kochte 6,5 h am Rückfluß. Die Reaktionsmischung wurde filtriert, das Filtrat eingeengt und der Rückstand aus n-Heptan umkristallisiert. Man erhielt 82 g N-[1-Cyanmethyl-2,2,6,6-tetramethyl-4-piperidinyl]-pelargonsäureamid vom Schmp. 68 ° C.

| | | | | |
|---|---|---|---|---|
| Ber.: | C 71,6 | H 11,0 | N 12,5 | O 4,8 |
| Gef.: | C 70,9 | H 10,9 | N 12,5 | O 5,4 |

b)
40 g des Produktes aus a) wurden wie in Beispiel 4b hydriert. Man filtrierte und engte ein. Der ölige Rückstand N-[1-β-Aminoethyl-2,2,6,6-tetramethyl-4-piperidinyl]-pelargonsäureamid konnte ohne Reinigung weiter umgesetzt werden.
c)
40 g des Produktes aus b) wurden mit Tetramethylolacetylendiharnstoff wie in Beispiel 4c zur Reaktion gebracht. Nach Einengen und Umkristallisation aus Acetonitril erhielt man 26,3 g Verbindung der Formel

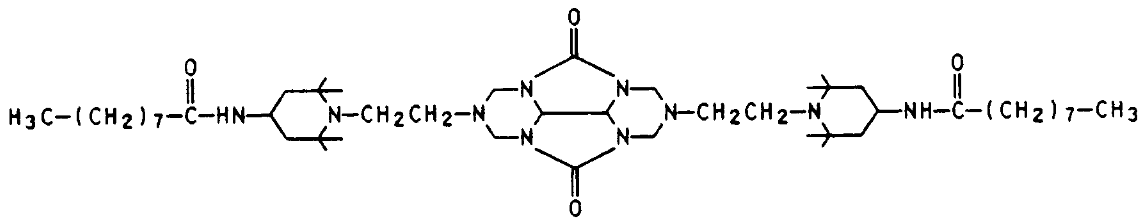

vom Schmp. 138 bis 139 °C.

Beispiel 7

a)
87 g Undecansäure-(2,2,6,6-tetramethyl-4-piperidinyl)-ester wurden analog Beispiel 6a umgesetzt. Nach Umkristallisation aus Petrolether erhielt man 58 g Undecansäure-(1-cyanmethyl-2,2,6,6-tetramethyl-4-piperidinyl)ester vom Schmp. 54 °C.
b)
40 g des Produktes aus a) wurden wie in Beispiel 4b hydriert. Nach Filtration und Einengen erhielt man 40 g Undecansäure-(1-β-aminoethyl-2,2,6,6-tetramethyl-4-piperidiny)-ester als öligen Rückstand, der zur weiteren Umsetzung geeignet war.
c)
20 g des Produktes aus b) wurden wie in Beispiel 4c mit Tetramethylolacetylendiharnstoff umgesetzt. Nach Einengen und Umkristallisation aus Acetonitril erhielt man 13 g Verbindung der Formel

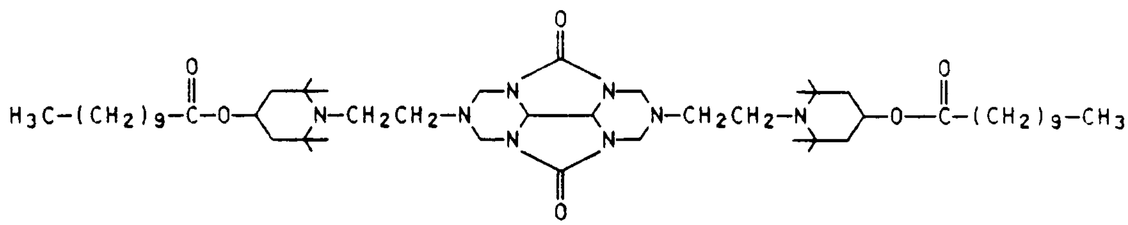

vom Schmp. 142 °C.

| | | | | |
|---|---|---|---|---|
| Ber.: | C 67,4 | H 10,2 | N 12,1 | O 10,4 |
| Gef.: | C 67,5 | H 10,3 | N 11,8 | O 10,2 |

Beispiel 8

a)
66 g Benzoesäure-(2,2,6,6-tetramethyl-4-piperidinyl)-ester wurden wie in Beispiel 6a umgesetzt. Man rotierte ein und kristallisierte aus iso-Propanol und Methanol um. Man erhielt 56,8 g Benzoesäure-(1-cyanmethyl-2,2,6,6-tetramethyl-4-piperidinyl)ester vom Schmp. 147 °C.

| | | | | |
|---|---|---|---|---|
| Ber.: | C 72,0 | H 8,0 | N 9,3 | O 10,7 |
| Gef.: | C 72,3 | H 8,1 | N 9,3 | O 10,7 |

b)
40 g des Produktes aus a) wurden wie in Beispiel 4b hydriert. Der nach Filtrieren und Einengen verbleibende ölige Rückstand Benzoesäure-(1-$\beta$-aminoethyl-2,2,6,6-tetramethyl-4-piperidinyl)ester wurde direkt in Beispiel 8c eingesetzt.
c)
40 g des Produktes aus b) wurden mit Tetramethylolacetylendiharnstoff analog Beispiel 4c umgesetzt und gereingt. Man erhielt 27,5 g der Verbindung der Formel

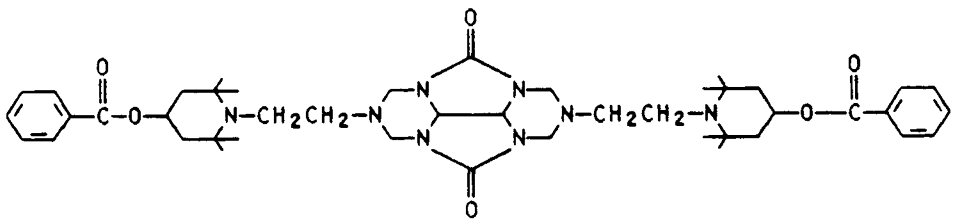

vom Schmp. 211 °C.

| | | | | |
|---|---|---|---|---|
| Ber.: | C 66,2 | H 7,8 | N 14,0 | O 12,0 |
| Gef.: | C 66,1 | H 8,0 | N 13,9 | O 11,9 |

Beispiel 9

a)
45 g 2,4-Dimethyl-furan-3-carbonsäure-(2,2,6,6-tetramethyl-4-piperidinyl)-ester wurden analog Beispiel 6a umgesetzt. Das Produkt wurde abgesaugt und aus iso-Propanol umkristallisiert. Man erhielt 37,9 g 2,4-Dimethyl-3-furan-3-carbonsäure-(1-cyanmethyl-2,2,6,6-tetramethyl-4-piperidinyl)ester vom Schmp. 156 °C.

| | | | | |
|---|---|---|---|---|
| Ber.: | C 67,9 | H 8,2 | N 8,8 | O 15,1 |
| Gef.: | C 67,7 | H 8,4 | N 8,7 | O 14,9 |

b)
30 g des Produktes aus a) wurden analog Beispiel 4b hydriert. Nach Filtrieren und Einengen erhielt man 2,4-Dimethyl-furan-3-carbonsäure-(1-$\beta$-aminoethyl-2,2,6,6-tetramethyl-4-piperidinyl)ester als öligen Rückstand, der ohne weitere Reinigung zur nächsten Umsetzung benutzt werden kann.
c)
28 g des Produktes aus b) wurden mit Tetramethylolacetylendiharnstoff wie in Beispiel 4c umgesetzt. Nach Einengen und Umkristallisation aus Acetonitril erhielt man 21,8 g der Verbindung der Formel

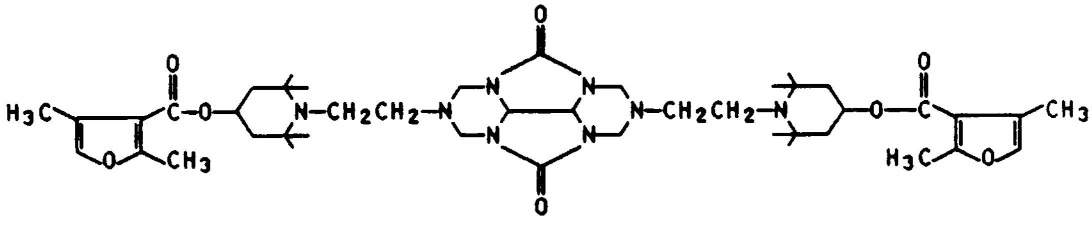

vom Schmp. 206 °C.

| | | | | |
|---|---|---|---|---|
| Ber.: | C 63,3 | H 8,0 | N 13,4 | O 15,3 |
| Gef.: | C 63,5 | H 8,0 | N 13,1 | O 15,1 |

Beispiel 10

a)
107 g 2,4,5-Trimethyl-furan-3-carbonsäure-(2,2,6,6-tetramethyl-4-piperidinyl)-ester wurden wie in Beispiel 6a umgesetzt. Nach Filtration und Einengen wurde mit Wasser aufgeschlämmt, der Rückstand abgesaugt und zweimal aus Methanol umkristallisiert. Man erhielt 57 g 2,4,5-Trimethyl-furan-3-carbonsäure-(1-cyanmethyl-2,2,6,6-tetramethyl-4-piperidinyl)-ester vom Schmp. 88 bis 89 °C.

| | | | | |
|---|---|---|---|---|
| Ber.: | C 68,7 | H 8,4 | N 8,4 | O 14,5 |
| Gef.: | C 68,7 | H 8,7 | N 8,1 | O 14,3 |

b)
40 g des Produktes aus a) wurden wie in Beispiel 4b hydriert. Der nach Filtrieren und Einengen erhältliche Rückstand wurde nach Trocknen an der Luft kristallin. Man erhielt 40 g 2,4,5-Trimethyl-furan-3-carbonsäure-(1-$\beta$-aminoethyl-2,2,6,6-tetramethyl-4-piperidinyl)ester vom Schmp. 77 bis 81 °C.

| | | | | |
|---|---|---|---|---|
| Ber.: | C 67,9 | H 9,5 | N 8,3 | O 14,3 |
| Gef.: | C 67,9 | H 9,6 | N 7,7 | O 14,6 |

c)
25 g des Produktes aus b) wurden mit Tetramethylolacetylendiharnstoff analog Beispiel 4c umgesetzt. Das aus der Reaktionsmischung ausgefallene Produkt wurde abgesaugt und mit iso-Butanol, Ethanol und Petrolether gewaschen. Man erhielt 18,7 g der Verbindung der Formel

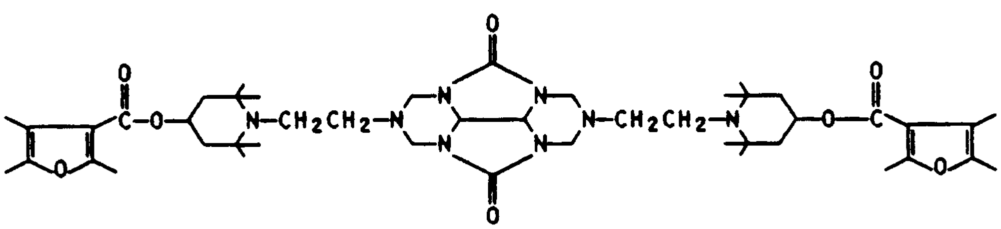

vom Schmp. 228 °C.

| | | | | |
|---|---|---|---|---|
| Ber.: | C 64,0 | H 8,1 | N 13,0 | O 14,8 |
| Gef.: | C 63,9 | H 8,6 | N 12,0 | O 15,3 |

Beispiel 11

a)
48 g Bis-[2,2,6,6-tetramethyl-4-piperidinyl]-sebacinsäurediester wurden analog Beispiel 6a umgesetzt. Nach Filtration und Einengen wurde der Rückstand aus iso-Propanol umkristallisiert und bei -20 °C abgesaugt. Man erhielt 41 g Bis-[1-cyanmethyl-2,2,6,6-tetramethyl-4-piperidinyl]-sebacinsäurediester vom Schmp. 91 °C.

| | | | | |
|---|---|---|---|---|
| Ber.: | C 68,8 | H 9,7 | N 10,0 | O 11,5 |
| Gef.: | C 68,8 | H 9,9 | N 10,0 | O 11,6 |

b)

34 g des Produktes aus a) wurden wie in Beispiel 4b hydriert. Der nach dem Filtrieren und Einengen erhaltene ölige Rückstand (Bis-[1-β-aminoethyl-2,2,6,6-tetramethyl-4-piperidinyl)sebacinsäurediester) kann direkt zur weiteren Umsetzung verwendet werden.

c)

27,5 g des Produktes aus b) wurden mit 25,1 g einer 50 %igen wäßrigen Lösung von Tetramethylolacetylendiharnstoff analog Beispiel 4c umgesetzt. Die Lösung wurde eingeengt, der Rückstand mit Eiswasser verrührt, abgesaugt und mit Wasser gewaschen. Man verrührte mit Petrolether, saugte ab, wusch mit Petrolether nach und trocknete an der Luft. Man erhielt 34 g der Verbindung der Formel

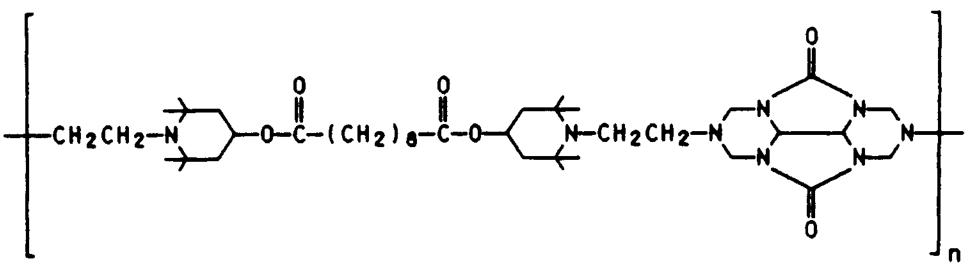

vom Glaspunkt 92 °C und Zersetzungspunkt 118 bis 120 °C. Die mittlere Molmasse (dampfdruckosmometrisch in Chloroform) betrug 6120 g/mol.

Beispiel 12

20 g des Produktes aus Beispiel 1 wurden in 100 ml Pyridin gelöst. Man tropfte 6,2 g Adipinsäuredichlorid zu, rührte 4 h bei Raumtemperatur und 11 h bei 50 bis 55 °C. Man verrührte mit Petrolether, saugte ab und behandelte den Rückstand mit 15 %iger, kalter Natronlauge. Das abgesaugte Produkt wurde mit Wasser neutral gewaschen und in heißem iso-Butanol gelöst. Nach 12 h wird das iso-Butanol vom ausgefallenen Niederschlag abdekantiert, der Rückstand mit Petrolether verrührt und abgesaugt. Man erhielt 8 g der Verbindung der Formel

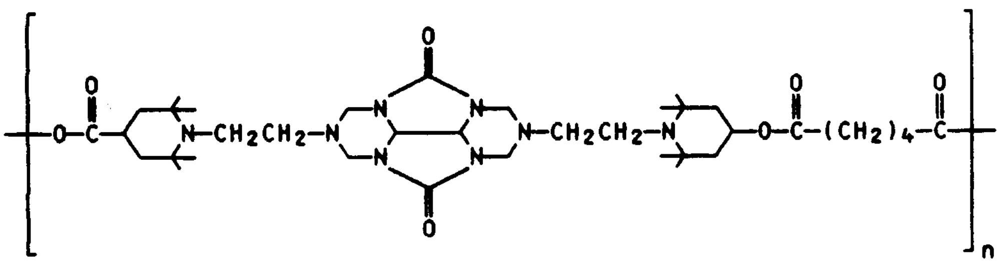

vom Schmp. 206 bis 207 °C. Die mittlere Molmasse (dampfdruckosmometrisch in Chloroform) betrug 1530 g/mol.

Beispiel 13

20 g des Produktes aus Beispiel 1 in 100 ml Pyridin wurden mit 16 g Pivalinsäurechlorid versetzt und 17 h bei Raumtemperatur gerührt. Man saugte ab, wusch mit Petrolether und rührte mit 15 %iger Natronlauge 10 min. auf. Die Mischung wurde mit n-Butanol extrahiert, die n-Butanolphase mit Wasser gewaschen und eingeengt. Nach Umkristallisation des Rückstandes aus Acetonitril erhielt man 14,3 g der Verbindung der Formel

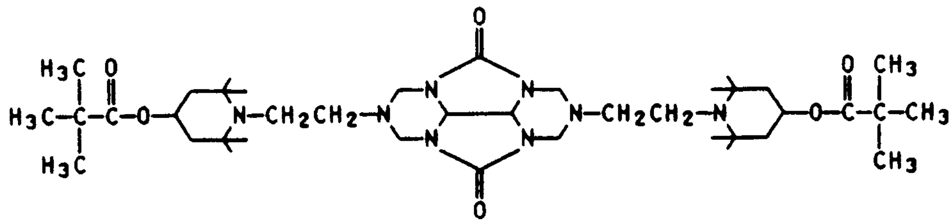

vom Schmp. 210 °C.

| | | | | |
|---|---|---|---|---|
| Ber.: | C 64,0 | H 8,3 | N 14,9 | O 12,8 |
| Gef.: | C 63,3 | H 9,2 | N 14,4 | O 12,3 |

Anwendungsbeispiel 1

Stabilisierung von Polyurethan
Herstellung der Belichtungsproben aus Polyurethan
Eine Polyolkomponente der Zusammensetzung
41,9 Teile eines Polyetherols der OH-Zahl von 29,0, das durch Addition von Propylenoxid und Ethylenoxid an Propylenglykol erhalten wurde und ungefähr 84 % primäre Hydroxylgruppen besitzt und 42,5 Teile eines Polyetherols der OH-Zahl von 27,0, das durch Addition von Propylenoxid und Ethylenoxid an Trimethylolpropan erhalten wurde und ungefähr 88 % primäre Hydroxylgruppen besitzt und 8,1 Teile 1,4-Butandiol und 1,724 Teile 25 %ige Lösung von Diazabicyclooctan in 1,4-Butandiol und 0,016 Teile Dibutylzinndilaurat und 0,1 Teile des Siliconstabilisators OS 710 der Firma Bayer und 5,49 Teile Frigen 11 und 0,17 Teile Wasser wurde mit der unten angegebenen Stabilisatormischung versetzt und im Mischungsverhältnis 100:48,5 mit einem 23,0 %-isocyanatgruppenhaltigen Prepolymeren bei 25 °C Komponenten-und Werkzeugtemperatur zu Prüfplatten verschäumt. Das NCO-Prepolymer wurde hergestellt aus 87,17 Teilen 4,4'-Diphenylmethandiisocyanat und 4,83 Teilen eines Polyetherols mit der OH-Zahl von 250, das durch Addition von Propylenoxid an Propylenglykol erhalten wurde und 8,0 Teile Dipropylenglykol.

Die Proben wurden im Xenotest®450 belichtet und an den Proben der Yellowness Index (YI) gemäß ASTM D 1925 bestimmt. Die Ergebnisse sind nachfolgend angegeben.

| Stabilisator-Mischung | Konz. (Gew.-%) | Yellowness-Index nach ASTM D 1925 im Xenotest 450 nach 0 h | 48 h |
|---|---|---|---|
| Beispiel 7c | 0,5 | | |
| Komponente 1 | 0,5 | 7,60 | 22,51 |
| Komponente 2 | 0,25 | | |
| Komponente 3 | 0,5 | | |
| Komponente 1 | 0,5 | 6,52 | 27,01 |
| Komponente 2 | 0,25 | | |

Komponente 1 =

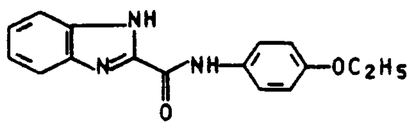

Komponente 2 = Mischung aus α-Tocopherol und Tri(nonylphenyl)-phosphit im Verhältnis 1:10.

Komponente 3 =

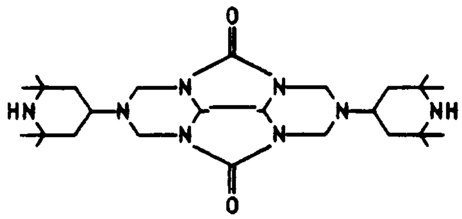

Verbindungen gemäß Komponente 3 sind aus der EP-A 213 570 bekannt.

Anwendungsbeispiel 2

Stabilisierung von Polypropylen

a)

0,25 Teile der Verbindung des entsprechenden Beispiels werden in 100 Teile Polypropylen (1320 H der Fa. BASF) durch zweimaliges Extrudieren bei 220 °C eingearbeitet und zu 200 µm dicken Platten gepreßt. Nach 14-tägiger Lagerung im Dunkeln bei 25 °C zeigt die Oberfläche der Platten keinen Belag.

b)

Die nach a) hergestellten Platten werden in einem Xenotest®1200-Prüfgerät auf ihre Bewitterungsstabilität getestet. Die Alterung wird durch Messen der "CO-Zahl" nach bestimmten Zeitintervallen bestimmt. Der Beginn der Versprödung wird mechanisch ermittelt. Die Testergebnisse sind in untenstehender Tabelle zusammengefaßt.

CO-Zahlen bei Belichtung in einem Xenotest®1200-Schnellbewitterungs-Prüfgerät (Polypropylen)

| Verbindung | Belichtungszeit in (h) | | |
|---|---|---|---|
| | 1000 | 2000 | 3000 |
| Beispiel 1 | 4,22 | 6,89 | spröde |
| Beispiel 2 | 4,69 | 5,55 | 7,00 |
| Beispiel 3 | 5,25 | 5,50 | |
| Komponente 3 | 5,80 | 13,09 | 21,0 |

## Patentansprüche

**1.** Verbindungen der allgemeinen Formel I

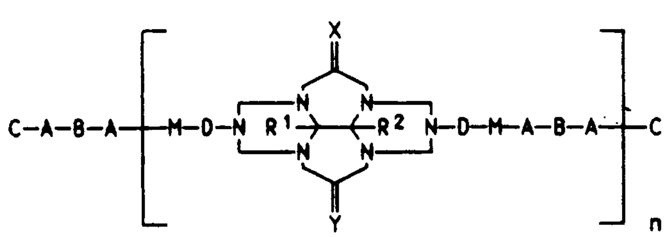 (I)

worin

n für eine ganze Zahl von 1 bis 70 steht,

$R^1$ und $R^2$ unabhängig voneinander Wasserstoff, $C_1$-$C_6$-Alkyl, $C_7$-$C_{12}$-Aralkyl, Aryl oder Carbonester bedeuten oder zusammen eine Tetra-, Penta- oder Hexamethylengruppierung darstellen, oder gemeinsam für einen gegebenenfalls durch $C_1$-$C_6$-Alkyl, $C_1$-$C_6$-Alkoxy, $C_2$-$C_{12}$-Carbonester, $C_1$-$C_{12}$-C-Acyl, Halogen, Nitro, Carboxyl, Amino, Hydroxy, -SH, -S-$C_1$-$C_4$-Alkyl, Phenyl, $C_2$-$C_6$-Alkenyl, (Poly)ethoxy, (Poly)amino substituierten Rest der Formel

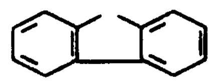

stehen,

X und Y unabhängig voneinander Sauerstoff, Schwefel oder $NR^7$ darstellen, wobei $R^7$ für Wasserstoff, $C_1$-$C_8$-Alkyl, oder $C_7$-$C_{12}$-Aralkyl steht,

M für

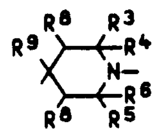

steht, worin $R^3$, $R^4$, $R^5$ und $R^6$ gleich oder verschieden sind und jeweils Alkyl bedeuten, oder worin $R^3$ und $R^4$ sowie $R^5$ und $R^6$ zusammen mit dem Kohlenstoffatom, an das sie gebunden sind, einen 5- oder 6-gliedrigen Ring bilden, und worin $R^8$ für Wasserstoff oder Alkyl steht, oder mit dem zugehörigen Kohlenstoffatom eine
$>C=O$ Gruppe
bildet, und worin $R^9$ Wasserstoff darstellt oder eine weitere Bindung zu einem spiroverknüpften Brückenglied -B- (-A- bedeutet in diesem Fall eine direkte Bindung) bedeutet,

D jeweils gleich oder verschieden ist und eine Gruppe-$(-CH_2-)_m$ darstellt, worin m eine Zahl von 1 bis 20 bedeutet, oder, falls der Rest M über das Stickstoffatom mit -A- verbunden ist, auch eine direkte Bindung sein kann,
wobei mindestens eine Gruppierung -M-D- im Molekül vorliegt und in dieser der Rest -M- über sein Stickstoffatom mit -D- verbunden ist.

A jeweils gleich oder verschieden ist und für Sauerstoff, gegebenenfalls einfach durch $C_1$-$C_6$-Alkyl, $C_1$-$C_{12}$-C-Acyl oder Acylgruppen der Formel $CO-(CH_2)_m-H$, wobei m eine Zahl von 0 bis 20 ist, substituierten Stickstoff, Carboxyl, Carbonyl, Sulfonyl, Sulfonamido, eine Gruppe der Formeln

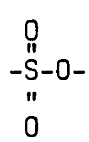 , 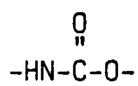 oder 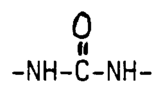

oder eine direkte Bindung steht,

B ein Brückenglied der Formeln

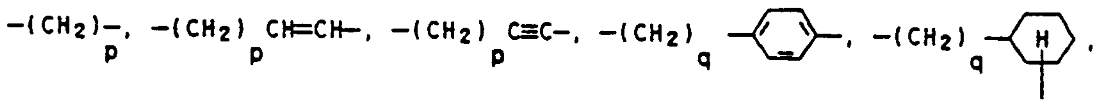

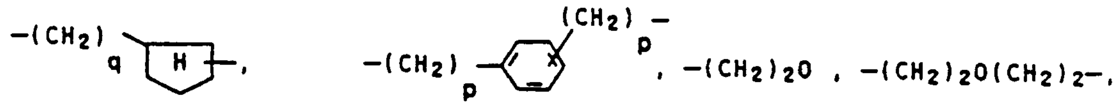

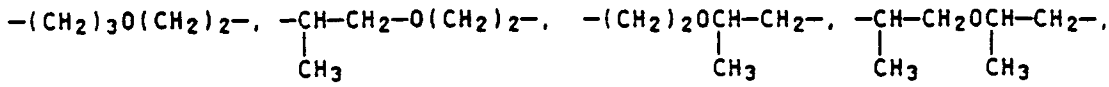

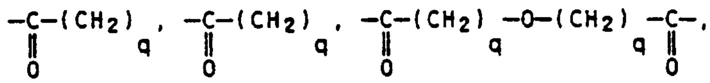

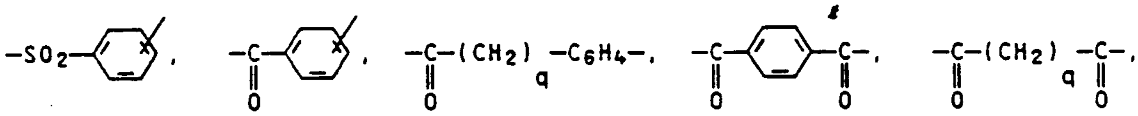

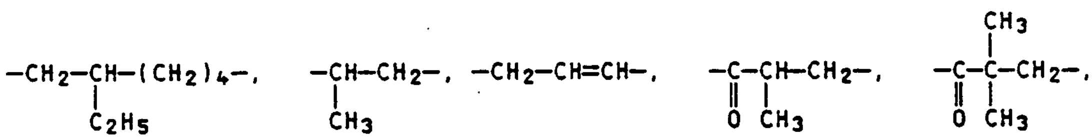

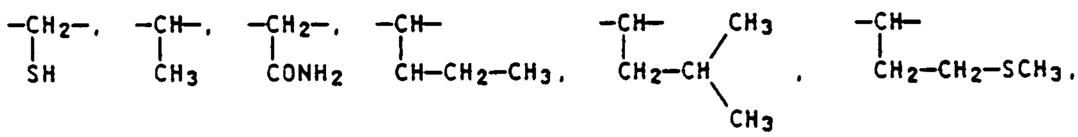

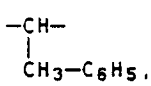, 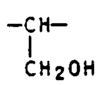, 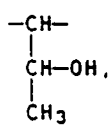, 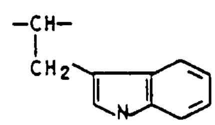, 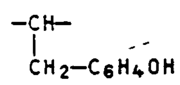, 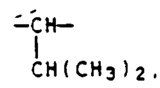,

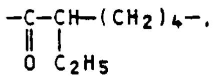, 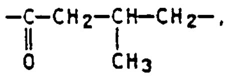, 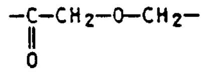 oder 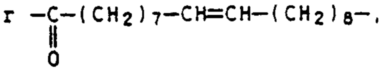,

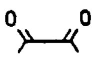, 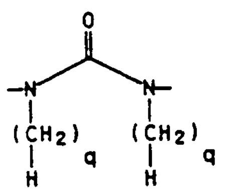, 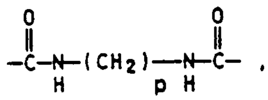,

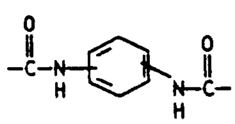, 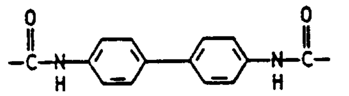,

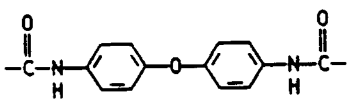, 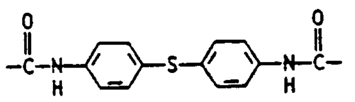,

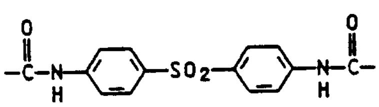, 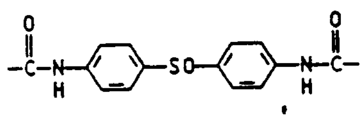,

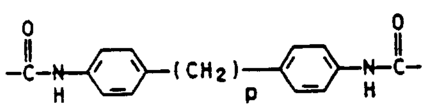, 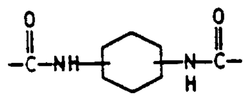,

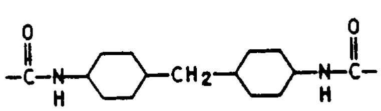, 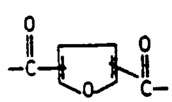, 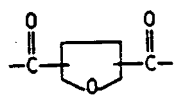,

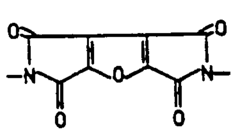, 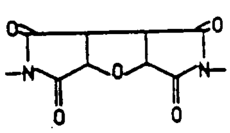, 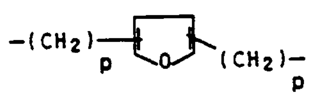,

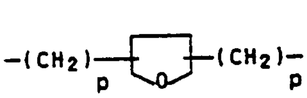, 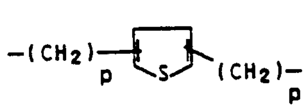, 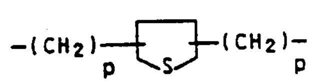,

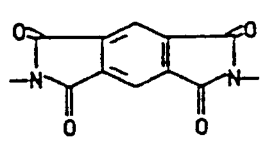, 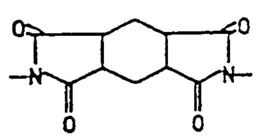,

wobei p = 1 bis 20 und q = 0 bis 20 ist,

und wobei das Brückenglied B mit dem Rest -M- auch durch eine Spiroverknüpfung der Struktur

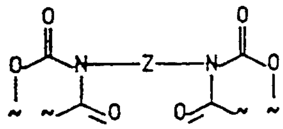

(Spiroverknüpfung an -M-) (Spiroverknüpfung an -M-) verbunden sein kann, worin Z ein Brückenglied darstellt und für $-(CH_2)_k-$ (k = 0 bis 20),

Phenylen, 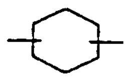, Xylylen, 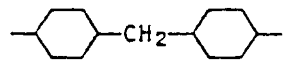 oder 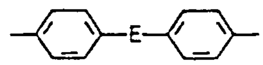

(E = O, N, S $SO_2$, SO, $-CH_2-$, $-CH_2-CH_2-$, direkte Bindung) steht, oder eine direkte Bindung bedeutet, und

C für Wasserstoff, $C_1$-$C_{20}$-C-Acyl, Chlor, Brom, Iod, Hydroxy, Amino, substituiertes Amino der Formel $H-(CH_2)_r-NH-$ (r = 0 bis 20), Alkoxy der Formel $H-(CH_2)_r-O-$ (r = 0 bis 20), Carboxyl, Carbonester, Cyan, Sulfonamido, Carbonyl-Gruppierungen der Formel

$H-(CH_2)_r-CO-$, 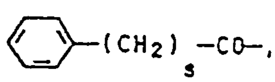, 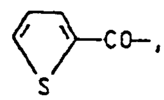,

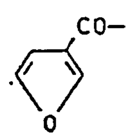, 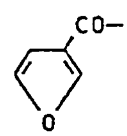, 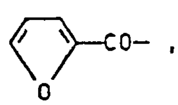, wobei r = 0 bis 20 und s = 1 bis 5 ist,

eine Harnstoffgruppierung der Formel

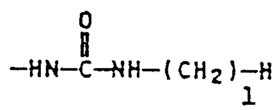, 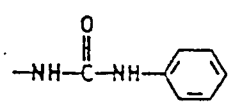, 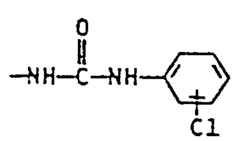,

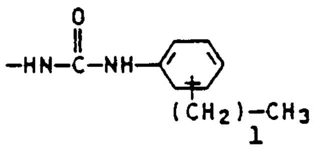 , 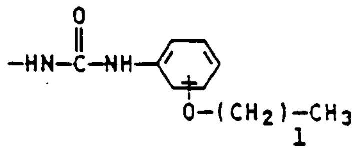 ,

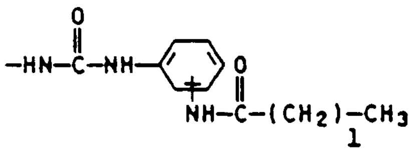 , 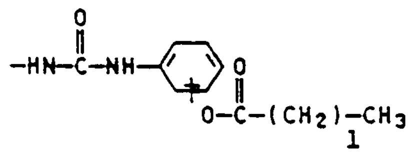 ,

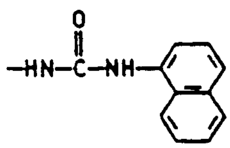 , 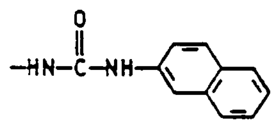 ,

wobei l = 0 bis 22 ist, oder eine Urethangruppe der Formel

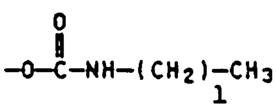 , 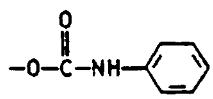 , 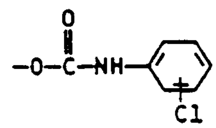 ,

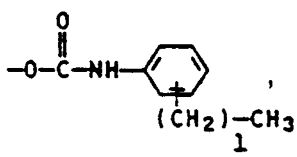 , 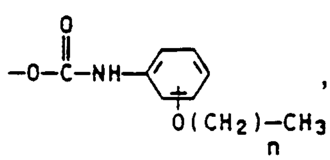 , 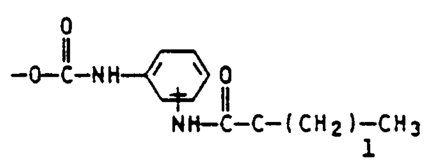

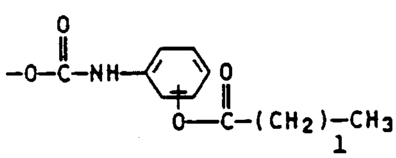 , 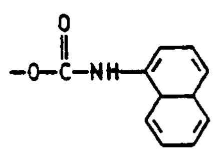 ,

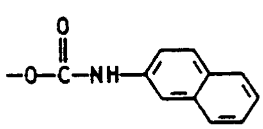 (l = 0 bis 22),

oder eine Aryl- oder Heteroarylcarbonylgruppierung der Formel

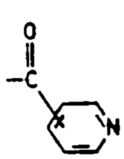 , 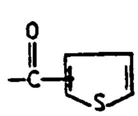 , 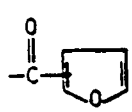 , 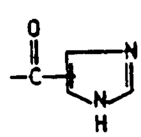 , 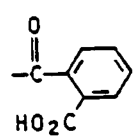 ,

steht, oder eine Gruppierung der Formel

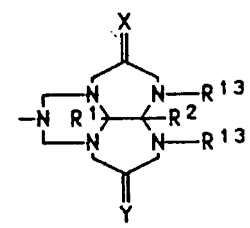

darstellt, worin die Reste $R^{13}$ gleich oder verschieden sind und für Wasserstoff oder $CH_2OR^{14}$ stehen, worin $R^{14}$ Wasserstoff oder einen $C_1$-$C_{22}$-Alkylrest, der durch Ethersauerstoff, Stickstoff oder Schwefel unterbrochen sein kann, bedeutet,
oder worin die Reste $R^{13}$ gemeinsam für eine Gruppierung

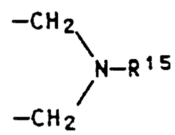

stehen können, worin $R^{15}$ die Bedeutungen [1)] $C_1$-$C_{22}$-Alkyl, das durch Ethersauerstoff, Schwefel oder Stickstoff unterbrochen sein kann, [2)] $C_1$-$C_{22}$-Alkenyl, [3)] $C_3$-$C_{12}$-Cycloalkyl, [4)] gegebenenfalls substituiertes $C_7$-$C_{12}$-Aralkyl, [5)] $C_3$-$C_{12}$-Alkinyl, [6)] nichtaromatischer Heterocyclus, [7)] $C_1$-$C_{22}$-Alkyl oder Aralkyl, die einen Heterocyclus enthalten oder [8)] $C_1$-$C_{22}$-Alkyl, das Hydroxy, Thiol, Cyan, Carboxyl, Carbonester, Carbamoyl, welches durch $C_1$-$C_6$-Alkyl, $C_1$-$C_6$-Alkoxy, $C_2$-$C_{12}$-Carbonester, $C_1$-$C_{12}$-C-Acyl, Halogen, Nitro, Carboxyl, Amino, Hydroxy, -SH, -S-$C_1$-$C_4$-Alkyl, Phenyl, $C_2$-$C_6$-Alkenyl, (Poly)ethoxy oder (Poly)amino substituiert sein kann, Brom, Chlor, Iod, Sulfon, Sulfonoxid, Sulfonyl, Sulfonamid, welches durch $C_1$-$C_6$-Alkyl, $C_1$-$C_6$-Alkoxy, $C_2$-$C_{12}$-Carbonester, $C_1$-$C_{12}$-C-Acyl, Halogen, Nitro, Carboxyl, Amino, Hydroxy, -SH, -S-$C_1$-$C_4$-Alkyl, Phenyl, $C_2$-$C_6$-Alkenyl, (Poly)ethoxy oder (Poly)amino substituiert sein kann, einen obengenannten Urethanrest oder Harnstoffres enthält, besitzt,
oder worin in der allgemeinen Formel (I) die Gruppierung -M-A-B-A-C für eine Gruppe der allgemeinen Formeln

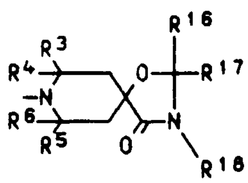 , 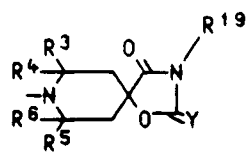

oder 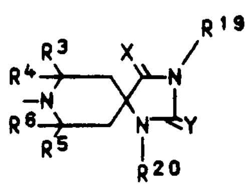 steht,

worin

| | |
|---|---|
| $R^3$ bis $R^6$ | sowie X und Y die oben angegebenen Bedeutungen besitzen, |
| $R^{16}$ und $R^{17}$ | unabhängig voneinander Wasserstoff, gegebenenfalls durch Ethersauerstoff, Schwefel oder Stickstoff unterbrochenes oder durch Cyan-, Carboxyl-, Carbamoyl-, Carbonester- oder Ketogruppen substituiertes $C_1$-$C_{22}$-Alkyl, $C_3$-$C_{12}$-Cycloalkyl, Bicycloalkyl, Tricycloalkyl, gegebenenfalls durch Chlor, $C_1$-$C_{22}$-Alkyl oder $C_1$-$C_{22}$-Alkoxy substituiertes Aryl, oder $C_7$-$C_{22}$-Aralkyl darstellen, oder worin |
| $R^{16}$ und $R^{17}$ | zusammen mit dem sie bindenden Kohlenstoffatom eine $C_5$-$C_{18}$-Cycloalkylgruppe, die durch bis zu 4 $C_1$-$C_4$-Alkylgruppen substituiert sein kann oder eine Gruppe der Formel |

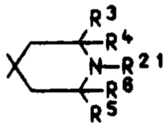

bilden, worin

$R^{21}$ Wasserstoff, $C_1$-$C_{22}$-Alkyl, das durch Ethersauerstoff, Schwefel oder Stickstoff unterbrochen oder durch Cyan, Hydroxyl, Thiol, Carboxyl-, Carbamoyl- oder Carbonestergruppen substituiert sein kann, $C_1$-$C_{22}$-Alkenyl oder $C_1$-$C_{22}$-C-Acyl bedeutet, und $R^3$, $R^4$, $R^5$, $R^6$ die vorstehenden Bedeutungen besitzen,

$R^{18}$ Wasserstoff, $C_1$-$C_{22}$-Alkyl, das durch Ethersauerstoff, Schwefel oder Stickstoff unterbrochen oder durch Cyan, Carboxyl-, Carbamoyl, Carbonester, Hydroxy-, Thiol- oder Aminogruppen substituiert sein kann oder $C_1$-$C_{22}$-C-Acyl,

$R^{19}$ Wasserstoff, $C_1$-$C_{22}$-Alkyl, das durch Ethersauerstoff, Schwefel oder Stickstoff unterbrochen oder durch Cyan- oder Carbonestergruppen substituiert sein kann, Aryl, das gegebenenfalls durch Chlor, $C_1$-$C_{22}$-Alkyl oder $C_1$-$C_{22}$-Alkoxy substituiert sein kann, $C_7$-$C_{22}$-Aralkyl oder $C_3$-$C_{12}$-Cycloalkyl und

$R^{20}$ Wasserstoff oder gegebenenfalls verzweigtes Alkyl bedeuten,

sowie die Säureadditionssalze und Hydrate dieser Verbindungen.

**2.** Verbindungen nach Anspruch 1, dadurch gekennzeichnet, daß n für 1 bis 20 steht.

**3.** Verbindungen nach Anspruch 1 oder 2, dadurch gekennzeichnet, daß $R^1$ und $R^2$ ausgewählt sind unter Wasserstoff, Methyl und Phenyl.

**4.** Verbindungen nach einem der Ansprüche 1 bis 3, dadurch gekennzeichnet, daß $R^3$, $R^4$, $R^5$ und $R^6$ für Methyl stehen.

**5.** Verbindungen nach einem der Ansprüche 1 bis 4, dadurch gekennzeichnet, daß X und Y Sauerstoff bedeuten.

**6.** Verbindungen nach einem der Ansprüche 1 bis 5, dadurch gekennzeichnet, daß -A- für Sauerstoff oder einmal acylierten Stickstoff steht.

**7.** Verbindungen nach einem der Ansprüche 1 bis 6, dadurch gekennzeichnet, daß -B- eine direkte Bindung oder eine Akylengruppe ist.

**8.** Verbindungen nach einem der Ansprüche 1 bis 7, dadurch gekennzeichnet, daß -C- für Wasserstoff oder eine $C_1$-$C_{20}$-C-Acylgruppe steht.

**9.** Gemische von Verbindungen der allgemeinen Formel I gemäß einem der Ansprüche 1 bis 8.

**10.** Verbindungen der Formel

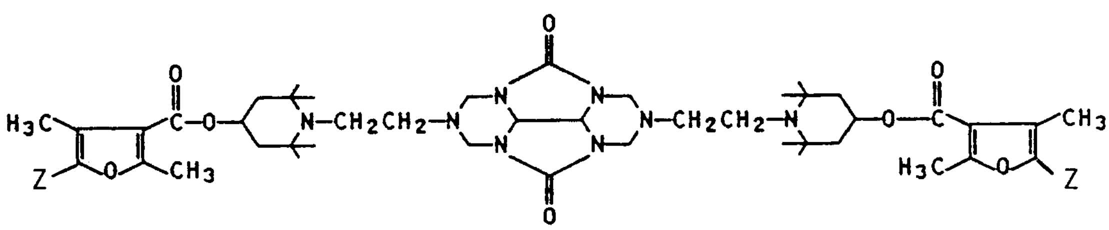

worin Z für Wasserstoff oder Methyl steht.

**11.** Verwendung der Verbindungen und deren Gemische nach einem der Ansprüche 1 bis 10 zum Stabilisieren von organischem Material.

**12.** Verwendung der Verbindungen und deren Gemische nach einem der Ansprüche 1 bis 10 zum Stabilisieren von Kunststoffen.

**13.** Verwendung nach Anspruch 12 zum Stabilisieren von Polyolefinen, Polyamiden und Lacken.

**14.** Verwendung nach einem der Ansprüche 11 bis 13 zum Licht- und Wärmeschutz oder als Metalldesaktivator.

**15.** Stabilisiertes organisches Material, enthaltend Verbindungen nach einem der Ansprüche 1 bis 10.

**16.** Verwendung nach Anspruch 12 zum Stabilisieren von Polyurethanen.

**Claims**

**1.** A compound of the general formula I

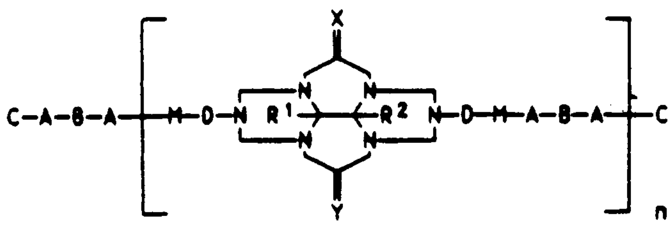

(I)

where

n is an integer from 1 to 70,

$R^1$ and $R^2$ are independently of each other hydrogen, $C_1$-$C_6$-alkyl, $C_7$-$C_{12}$-aralkyl, aryl or carboxylate or together a tetra-, penta- or hexamethylene group, or conjointly an unsubstituted or $C_1$-$C_6$-alkyl-, $C_1$-$C_6$-alkoxy-, $C_2$-$C_{12}$-carboxylate-, $C_1$-$C_{12}$-C-acyl-, halogen-, nitro-, carboxyl-, amino-, hydroxyl-, -SH-, -S-$C_1$-$C_4$-alkyl-, phenyl-, $C_2$-$C_6$-alkenyl-, (poly)ethoxy- or (poly)amino-substituted radical of the formula

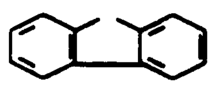

X and Y are independently of each other oxygen, sulfur or $NR^7$, where $R^7$ is hydrogen, $C_1$-$C_8$-alkyl or $C_7$-$C_{12}$-aralkyl,

M is

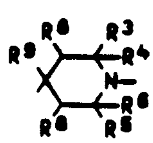

where $R^3$, $R^4$, $R^5$ and $R^6$ are identical or different and each is alkyl, or where $R^3$ and $R^4$ and also $R^5$ and $R^6$, together with the carbon atom to which they are bonded, form a 5- or 6-membered ring, and where $R^8$ is hydrogen or alkyl, or together with the associated carbon atom forms a
$>C=O$ group
and where $R^9$ is hydrogen or a further bond to a spiro-linked bridge member -B- (-A- is in this case a direct bond), the

D's are identical or different and each is a $(-CH_2-)_m$ group, where m is a number from 1 to 20, or, if the radical M is bonded to -A- via the nitrogen atom, can also be a direct bond, one or more -M-D- groups being present in the molecule where the radical -M- is bonded to -D- via its nitrogen atom, the

A's are identical or different and each is oxygen, nitrogen optionally monosubstituted by $C_1$-$C_6$-alkyl, $C_1$-$C_{12}$-C-acyl or acyl groups of the formula CO-$(CH_2)_m$-H, where m is from 0 to 20, carboxyl, carbonyl, sulfonyl, sulfonamido, a group of the formulae

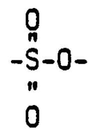 , 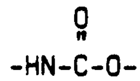 or 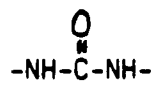

or a direct bond,

B is a bridge member of the formulae

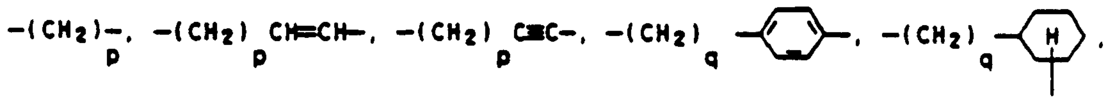

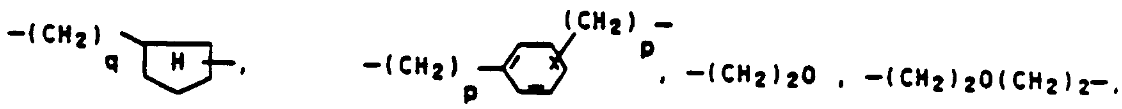

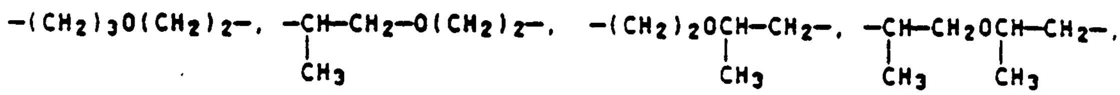

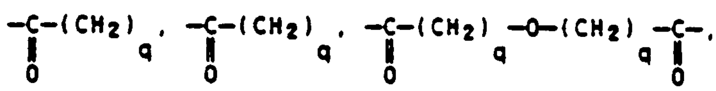

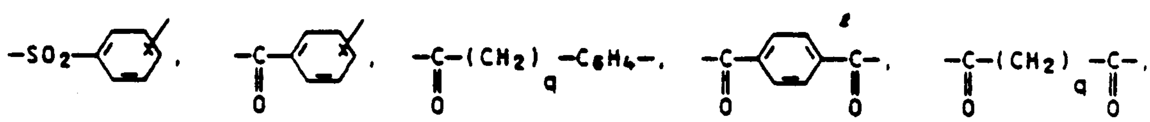

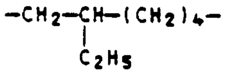 , 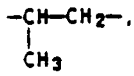 , 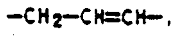 , 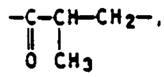 , 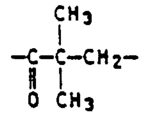 ,

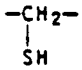 , 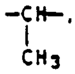 , 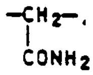 , 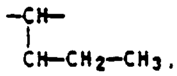 , 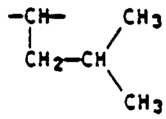 , 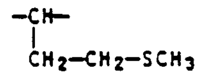 ,

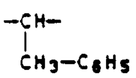 , 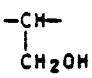 , 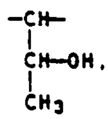 , 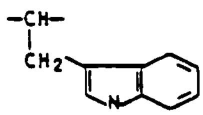 , 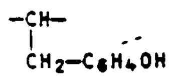 , 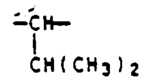 ,

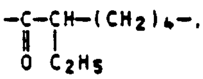 , 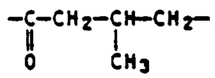 , 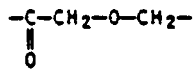 oder 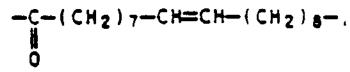 ,

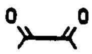 , 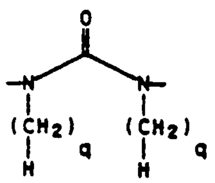 , 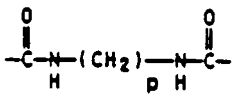 ,

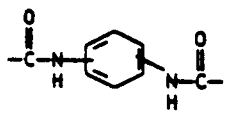 , 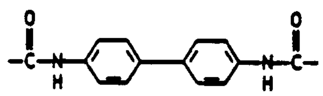 ,

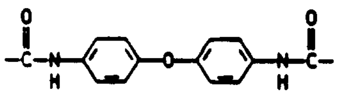 , 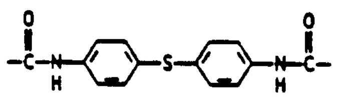 ,

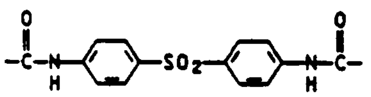 , 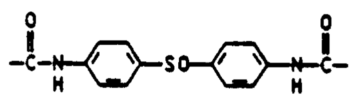 ,

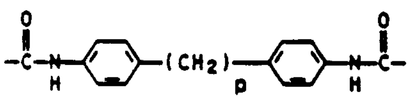 , 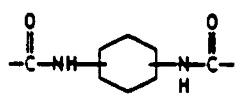 ,

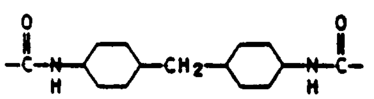 , 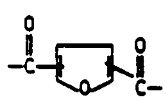 , 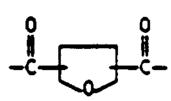 ,

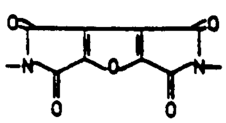 , 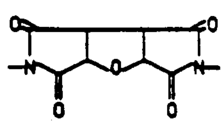 , 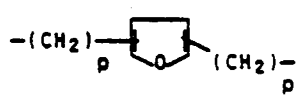 ,

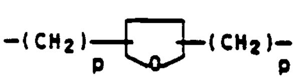 , 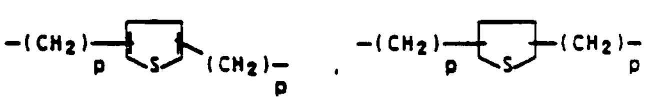

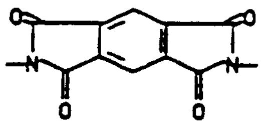,
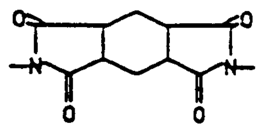,

where p is from 1 to 20 and q is from 0 to 20,
and where the bridge member B may also be linked to the radical -M- by a spiro linkage of the structure

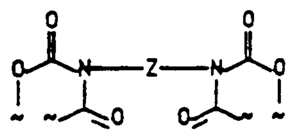

(spiro linkage to -M-)      (spiro linkage to -M-),

where Z is a bridge member and represents $-(CH_2)_k-$ (k = 0-20), phenylene,

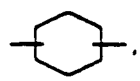,

xylylene

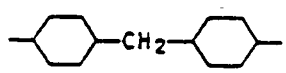

or

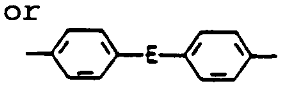

(E = O, N, S, $SO_2$, SO, $-CH_2-$, $-CH_2-CH_2-$, direct bond), or is a direct bond, and

C is hydrogen, $C_1$-$C_{20}$-C-acyl, chlorine, bromine, iodine, hydroxyl, amino, substituted amino of the formula $H-(CH_2)_r-NH-$ (r = 0-20), alkoxy of the formula $H-(CH_2)_r-O-$ (r = 0-20), carboxyl, carboxylate, cyano, sulfonamido, carbonyl groups of the formula

$H-(CH_2)_r-CO-$, 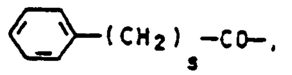, 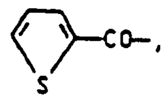,

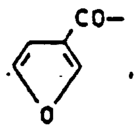, 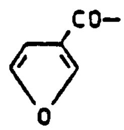, 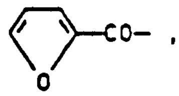, where r = 0-20 and s = 1-5,

a urea group of the formula

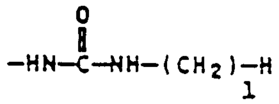, 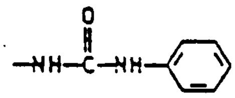, 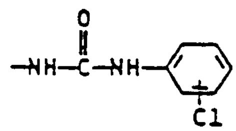,

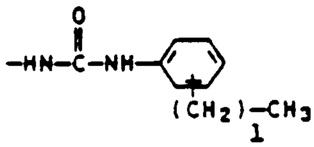, 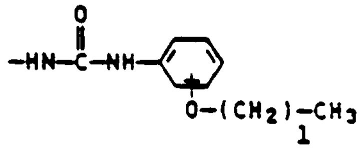,

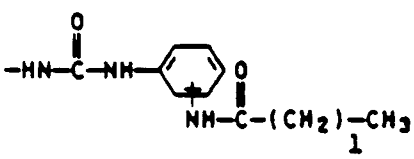, 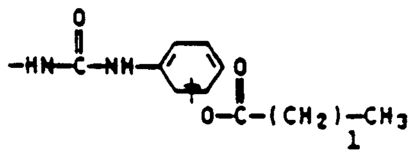,

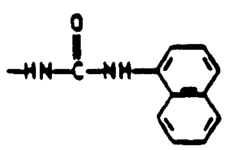, 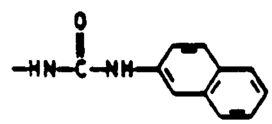,

where l = 0-22,
or a urethane group of the formula

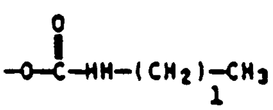, 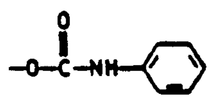, 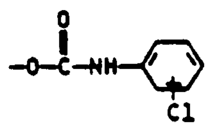,

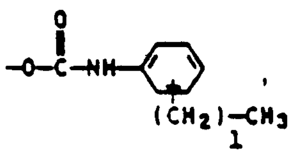, 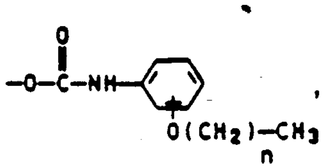, 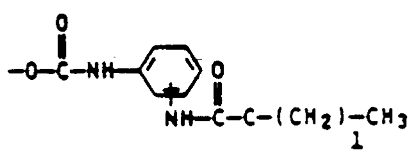

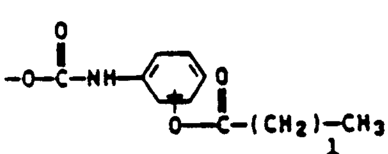, 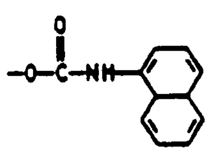,

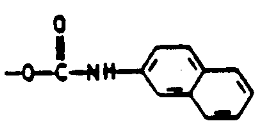

(l = 0-22)

or an aryl- or heteroaryl-carbonyl group of the formula

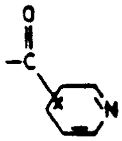 , 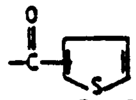 , 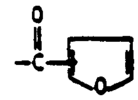 , 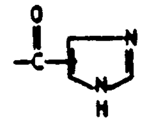 , 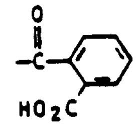 ,

or a group of the formula

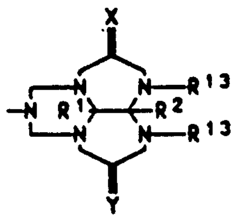

where the radicals $R^{13}$ are identical or different and each is hydrogen or $CH_2OR^{14}$, where $R^{14}$ is hydrogen or $C_1$-$C_{22}$-alkyl which may be interrupted by etheroxygen, nitrogen or sulfur,
or where the radicals $R^{13}$ together can be a group

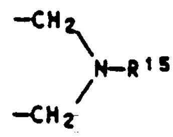

where $R^{15}$ has the meanings 1) $C_1$-$C_{22}$-alkyl, which may be interrupted by etheroxygen, sulfur or nitrogen, 2) $C_1$-$C_{22}$-alkenyl, 3) $C_3$-$C_{12}$-cycloalkyl, 4) optionally substituted $C_7$-$C_{12}$-aralkyl, 5) $C_3$-$C_{12}$-alkynyl, 6) nonaromatic heterocycle, 7) $C_1$-$C_{22}$-alkyl or aralkyl, which in each case contain a heterocycle, or 8) $C_1$-$C_{22}$-alkyl, which contains hydroxyl, thiol, cyano, carboxyl, carboxylate, carbamoyl, which may be $C_1$-$C_6$-alkyl-, $C_1$-$C_6$-alkoxy-, $C_2$-$C_{12}$-carboxylate-, $C_1$-$C_{12}$-C-acyl-, halogen-, nitro-, carboxyl-, amino-, hydroxyl-, SH-, -S-$C_1$-$C_4$-alkyl-, phenyl-, $C_2$-$C_6$-alkenyl-, (poly)ethoxy- or (poly)amino-substituted, bromine, chlorine, iodine, sulfone, sulfone oxide, sulfonyl, sulfonamide, which may be $C_1$-$C_6$-alkyl-, $C_1$-$C_6$-alkoxy-, $C_2$-$C_{12}$-carboxylate-, $C_1$-$C_{12}$-C-acyl-, halogen-, nitro-, carboxyl-, amino-, hydroxyl-, SH-, -S-$C_1$-$C_4$-alkyl-, phenyl-, $C_2$-$C_6$-alkenyl-, (poly)ethoxy- or (poly)amino-substituted, an abovementioned urethane radical or urea radical
or where in the general formula (I) the group -M-A-B-A-C- is a group of the general formula

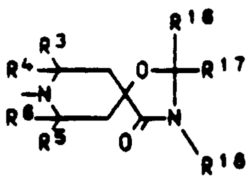 , 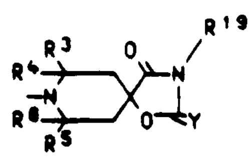

or

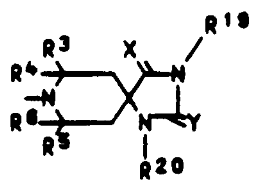

where

| | |
|---|---|
| $R^3$ to $R^6$ | and also X and Y are as defined above, |
| $R^{16}$ and $R^{17}$ | are independently of each other hydrogen, optionally etheroxygen-, sulfur- or nitrogen-interrupted or cyano-, carboxyl-, carbamoyl-, carboxylate- or keto-substituted $C_1$-$C_{22}$-alkyl, $C_3$-$C_{12}$-cycloalkyl, bicycloalkyl, tricycloalkyl, optionally chlorine-, $C_1$-$C_{22}$-alkyl- or $C_1$-$C_{22}$-alkoxy-substituted aryl, or $C_7$-$C_{22}$-aralkyl or where |
| $R^{16}$ and $R^{17}$ | together with the carbon atom joining them form a $C_5$-$C_{18}$-cycloalkyl group which |

may be substituted by up to 4 $C_1$-$C_4$-alkyl groups, or a group of the formula

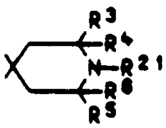

where $R^{21}$ is hydrogen, $C_1$-$C_{22}$-alkyl, which may be interrupted by etheroxygen, sulfur or nitrogen or substituted by cyano, hydroxyl, thiol, carboxyl, carbamoyl or carboxylate, $C_1$-$C_{22}$-alkenyl or $C_1$-$C_{22}$-C-acyl, and $R^3$, $R^4$, $R^5$ and $R^6$ have the abovementioned meaning,

$R^{18}$ is hydrogen, $C_1$-$C_{22}$-alkyl, which may be interrupted by etheroxygen, sulfur or nitrogen or substituted by cyano, carboxyl, carbamoyl, carboxylate, hydroxyl, thiol or amino, or $C_1$-$C_{22}$-C-acyl,

$R^{19}$ is hydrogen, $C_1$-$C_{22}$-alkyl, which may be interrupted by etheroxygen, sulfur or nitrogen or substituted by cyano or carboxylate, aryl, which can be optionally substituted by chlorine, $C_1$-$C_{22}$-alkyl or $C_1$-$C_{22}$-alkoxy, $C_7$-$C_{22}$-aralkyl or $C_3$-$C_{12}$-cycloalkyl and

$R^{20}$ is hydrogen or optionally branched alkyl,

and the acid addition salts and hydrates thereof.

**2.** A compound as claimed in claim 1, wherein n is 1-20.

**3.** A compound as claimed in claim 1 or 2, wherein $R^1$ and $R^2$ are selected from the group consisting of hydrogen, methyl and phenyl.

**4.** A compound as claimed in any of claims 1 to 3, wherein $R^3$, $R^4$, $R^5$ and $R^6$ are each methyl.

**5.** A compound as claimed in any of claims 1 to 4, wherein X and Y are each hydrogen.

**6.** A compound as claimed in any of claims 1 to 5, wherein -A- is oxygen or monoacylated nitrogen.

**7.** A compound as claimed in any of claims 1 to 6, wherein -B- is a direct bond or alkylene.

**8.** A compound as claimed in any of claims 1 to 7, wherein -C- is hydrogen or $C_1$-$C_{20}$-C-acyl.

**9.** A mixture of compounds of the general formula I as claimed in any of claims 1 to 8.

**10.** A compound of the formula

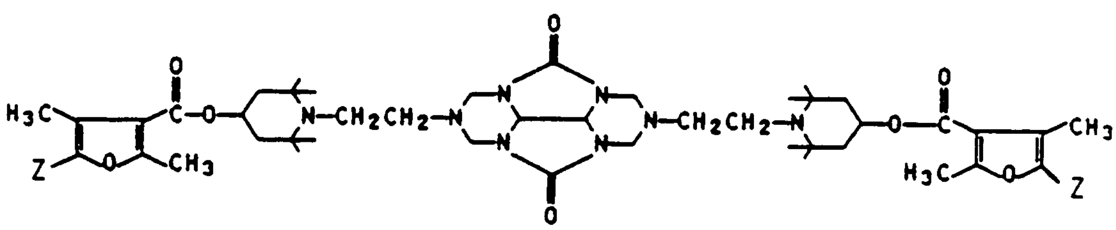

where Z is hydrogen or methyl.

**11.** The use of the compounds and the mixtures thereof as claimed in any of claims 1 to 10, for stabilizing organic material.

**12.** The use of the compounds and the mixtures thereof as claimed in any of claims 1 to 10, for stabilizing plastics.

**13.** Use as claimed in claim 12 for stabilizing polyolefins, polyamides and paints.

**14.** Use as claimed in any of claims 11 to 13 for light and heat stabilization or as metal deactivator.

**15.** A stabilized organic material containing a compound as claimed in any of claims 1 to 10.

**16.** Use of a compound as claimed in claim 12 for stabilizing polyurethane.

**Revendications**

**1.** Composés de formule générale I

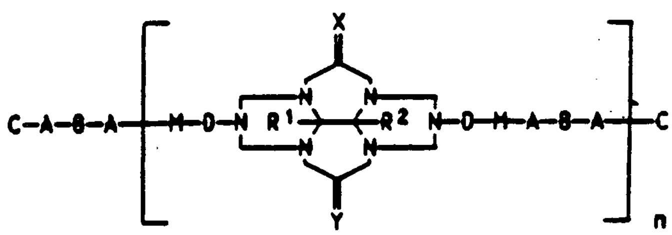

(I)

dans laquelle

n est mis pour un nombre entier de 1 à 70,

$R^1$ et $R^2$ désignent chacun, indépendamment l'un de l'autre, un atome d'hydrogène, un reste alkyle en $C_1$-$C_6$, aralkyle en $C_7$-$C_{12}$, aryle ou ester carboxylique, ou représentent ensemble un groupement tétra-, penta- ou hexaméthylène, ou sont mis en commun pour un reste de formule

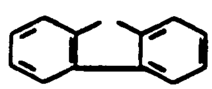

éventuellement substitué par un radical alkyle en $C_1$-$C_6$, alcoxy en $C_1$-$C_6$, ester carboxylique en $C_2$-$C_{12}$, C-acyle en $C_1$-$C_{12}$, halogéno, nitro, carboxyle, amino, hydroxy, -SH, -S-alkyle en $C_1$-$C_4$, phényle, alcényle en $C_2$-$C_6$, (poly)éthoxy ou (poly)amino,

X et Y représentent chacun, indépendamment l'un de l'autre, un atome d'oxygène, de soufre ou un groupement $NR^7$, $R^7$ étant mis pour un atome d'hydrogène, un reste alkyle en $C_1$-$C_8$ ou aralkyle en $C_7$-$C_{12}$,

M est mis pour

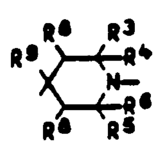

,

où $R^3$, $R^4$, $R^5$ et $R^6$ sont identiques ou différents et représentent chacun un reste alkyle, ou bien $R^3$ et $R^4$ d'une part et $R^5$ et $R^6$ d'autre part forment ensemble, avec l'atome de carbone auquel ils sont fixés, un cycle penta- ou hexagonal, $R^8$ est mis pour un atome d'hydrogène ou un reste alkyle ou bien forme, avec l'atome de carbone associé, un groupement $>C=O$, et $R^9$ représente un atome d'hydrogène ou une autre liaison pour un maillon de pontage spiro-enchaîné -B- (-A- représente dans ce cas une liaison directe),

D est chaque fois identique ou différent et représente un groupement $-(-CH_2-)_m$, dans lequel m est un nombre de 1 à 20, ou, dans le cas où le reste M est relié à -A- par

l'atome d'azote, il peut aussi être une liaison directe,
au moins un groupement -M-D- étant présent dans la molécule et, dans ce groupement, le reste -M- étant relié à -D- par son atome d'azote,

A est chaque fois identique ou différent et est mis pour un atome d'oxygène, d'azote éventuellement substitué une fois par un reste alkyle en $C_1$-$C_6$, C-acyle en $C_1$-$C_{12}$ ou par des groupements acyle de formule $CO-(CH_2)_m-H$ (m étant un nombre de 0 à 20), pour un radical carboxyle, carbonyle, sulfonyle, sulfonamido,
pour un groupement de formule

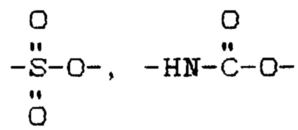 ou

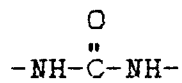

ou pour une liaison directe,

B représente un maillon de pontage de formule

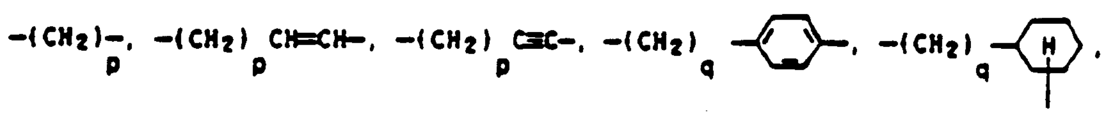

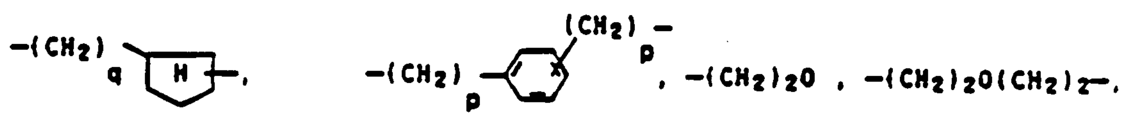

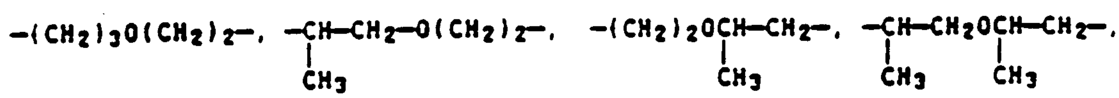

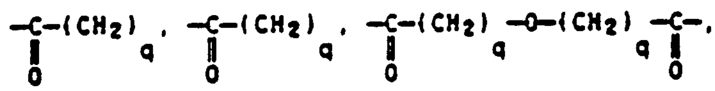

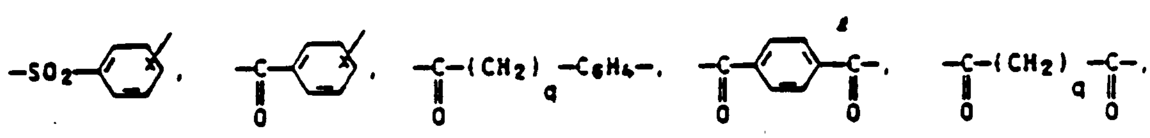

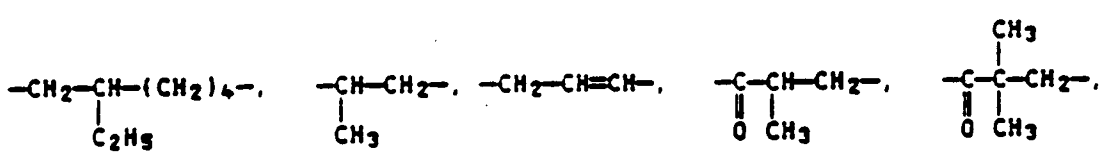

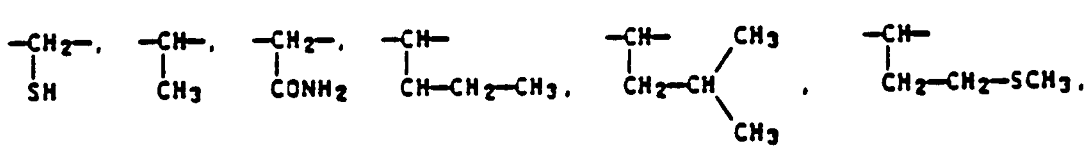

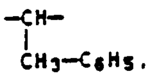 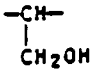 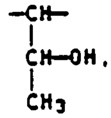 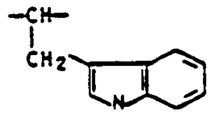 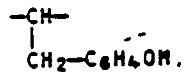 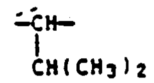

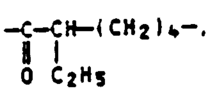 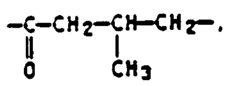 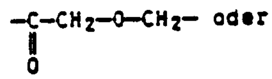 oder 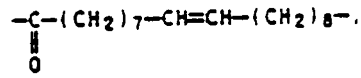

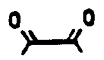 , 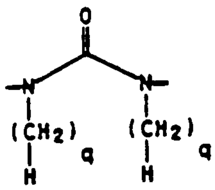 , 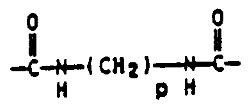 ,

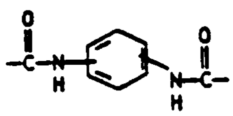 , 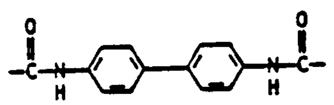 ,

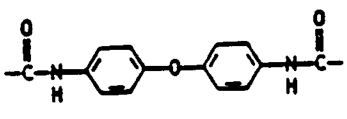 , 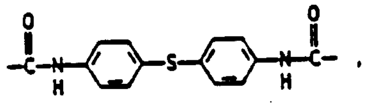 ,

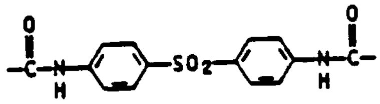 , 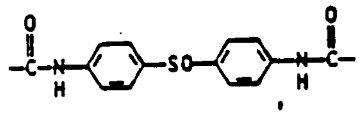 ,

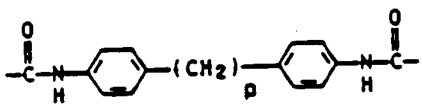 , 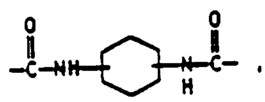 ,

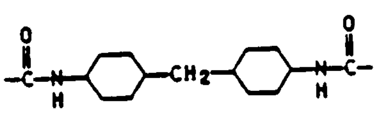 , 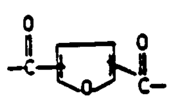 , 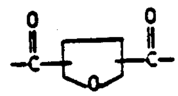 ,

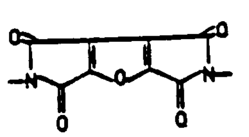 , 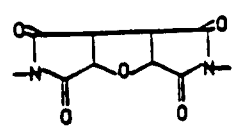 , 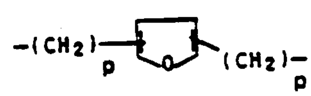 ,

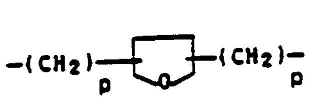 , 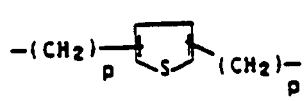 , 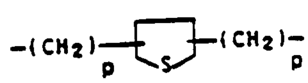 ,

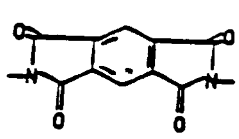 , 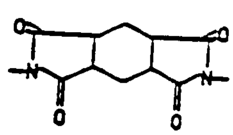 ,

avec p = 1 à 20 et q = 0 à 20,

le maillon de pontage B pouvant être également relié au reste -M- par un spiro-enchaînement de structure

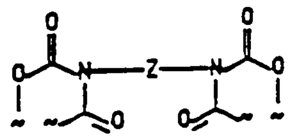

(spiro-enchaînement à -M-) (spiro-enchaînement à -M-)

dans laquelle Z représente un maillon de pontage et ~ est mis pour un radical $-(CH_2)_k-$ (k = 0 à 20), phénylène,

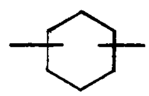

,

xylylène,

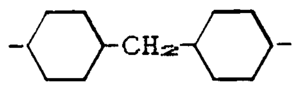

ou

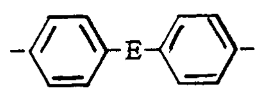

(E = O, N, S, $SO_2$, SO, $-CH_2-$, $-CH_2-CH_2-$, liaison directe),
ou il représente une liaison directe, et

C est mis pour un atome d'hydrogène, pour un radical C-acyle en $C_1$-$C_{20}$, chloro, bromo, iodo, hydroxy, amino, amino substitué de formule $H-(CH_2)_r-NH-$ (r = 0 à 20), alcoxy de formule $H-(CH_2)_r-O-$ (r = 0 à 20), carboxyle, ester carboxylique, cyano, sulfonamido, pour un groupement carbonyle de formule

$H-(CH_2)_r-CO-$, 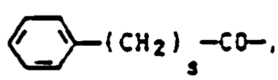 , 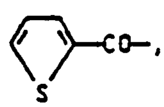

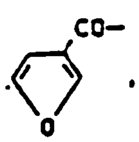 , 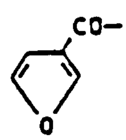 , 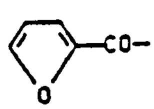 , avec r = 0 à 20 et s = 1 à 5,

pour un groupement urée de formule

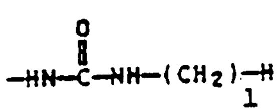 , 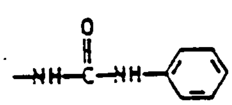 , 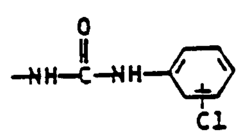 ,

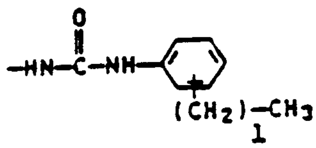 , 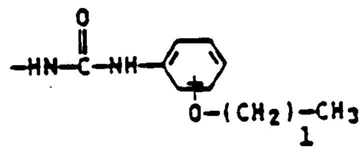

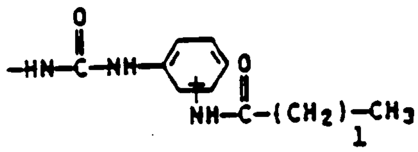 , 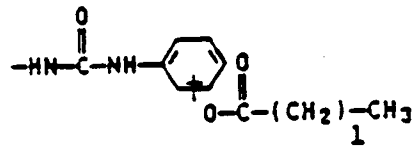

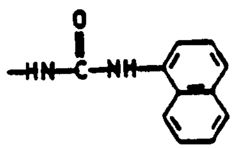 , 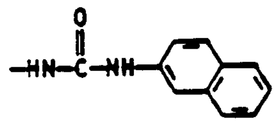 ,

avec l = 0 à 22,
pour un groupement uréthanne de formule

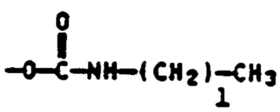 , 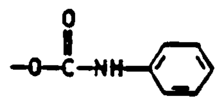 , 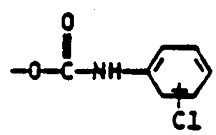 ,

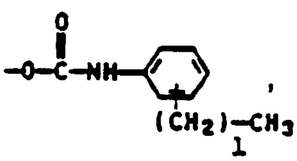 , 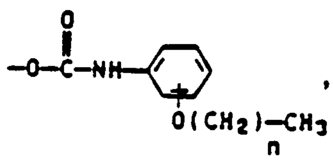 , 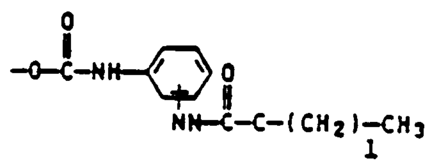

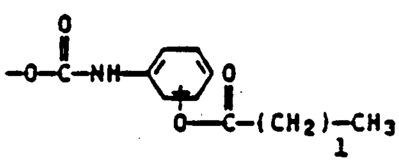 , 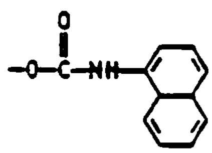 ,

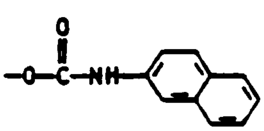 (l = 0 à 22),

ou pour un groupement aryl- ou hétéroarylcarbonyle de formule

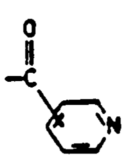 , 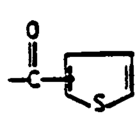 , 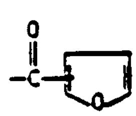 , 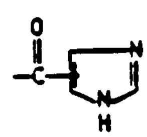 , 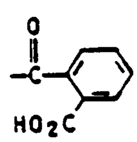 ,

ou il représente un groupement de formule

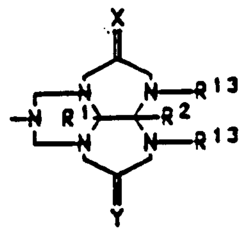

dans laquelle les restes $R^{13}$ sont identiques ou différents et sont mis pour des atomes d'hydrogène ou pour $CH_2OR^{14}$, où $R^{14}$ représente un atome d'hydrogène ou un reste alkyle en $C_1$-$C_{22}$ qui peut être interrompu par l'atome d'oxygène d'une fonction éther, d'azote ou de soufre,
ou dans laquelle les restes $R^{13}$ peuvent être mis en commun pour un groupement

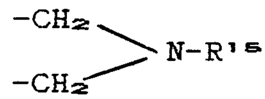

où $R^{15}$ a les significations suivantes: 1) alkyle en $C_1$-$C_{22}$ qui peut être interrompu par l'atome d'oxygène d'une fonction éther, de soufre ou d'azote; 2) alcényle en $C_1$-$C_{22}$; 3) cycloalkyle en $C_3$-$C_{12}$; 4) aralkyle en $C_7$-$C_{12}$ éventuellement substitué; 5) alcynyle en $C_3$-$C_{12}$; 6) hétérocycle non aromatique; 7) alkyle en $C_1$-$C_{22}$ ou aralkyle qui contiennent un hétérocycle; ou 8) alkyle en $C_1$-$C_{22}$ qui contient un reste hydroxy, thiol, cyano, carboxyle, ester carboxylique, carbamoyle [qui peut être substitué par un radical alkyle en $C_1$-$C_6$, alcoxy en $C_1$-$C_6$, ester carboxylique en $C_2$-$C_{12}$, C-acyle en $C_1$-$C_{12}$), halogéno, nitro, carboxyle, amino, hydroxy, -SH, -S-alkyle en $C_1$-$C_4$, phényle, alcényle en $C_2$-$C_6$, (poly)éthoxy ou (poly)amino], un reste bromo, chloro, iodo, sulfone, oxyde de sulfone, sulfonyle, sulfonamide [qui peut être substitué par un radical alkyle en $C_1$-$C_6$, alcoxy en $C_1$-$C_6$, ester carboxylique en $C_2$-$C_{12}$, C-acyle en $C_1$-$C_{12}$, halogéno, nitro, carboxyle, amino, hydroxy, -SH, -S-alkyle en $C_1$-$C_4$, phényle, alcényle en $C_2$-$C_6$, (poly)éthoxy ou (poly)amino], un reste uréthanne ou un reste urée sus-mentionné,
ou bien, dans la formule générale (I), le groupement -M-A-B-A-C- est mis pour un groupement de formule générale

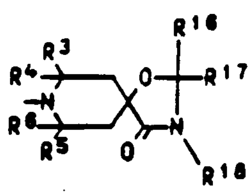

,

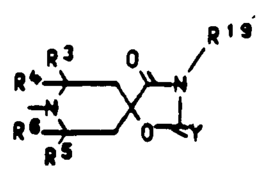

ou

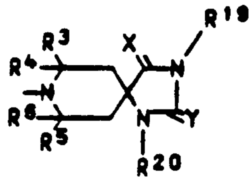

dans laquelle

| | |
|---|---|
| $R^3$ à $R^6$, | ainsi que X et Y ont les significations données ci-dessus, |
| $R^{16}$ et $R^{17}$ | représentent chacun, indépendamment l'un de l'autre, un atome d'hydrogène, un reste alkyle en $C_1$-$C_{22}$ éventuellement interrompu par l'atome d'oxygène d'une fonction éther, de soufre ou d'azote ou substitué par des groupements cyano, carboxyle, carbamoyle, ester carboxylique ou céto, un reste cycloalkyle en $C_3$-$C_{12}$, bicycloalkyle, tricycloalkyle, aryle éventuellement substitué par des radicaux chloro, |

alkyle en $C_1$-$C_{22}$ ou alcoxy en $C_1$-$C_{22}$, ou un reste aralkyle en $C_7$-$C_{22}$, ou dans laquelle

$R^{16}$ et $R^{17}$ forment ensemble, avec l'atome de carbone qui les relie, un groupement cycloalkyle en $C_5$-$C_{18}$ qui peut être substitué par jusqu'à 4 groupements alkyle en $C_1$-$C_4$, ou un groupement de formule

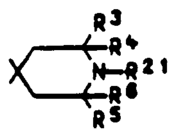

dans laquelle

$R^{21}$ représente un atome d'hydrogène, un reste alkyle en $C_1$-$C_{22}$, qui peut être interrompu par des atomes d'oxygène d'une fonction éther, de soufre ou d'azote ou être substitué par des groupements cyano, hydroxy, thiol, carboxyle, carbamoyle ou ester carboxylique, un reste alcényle en $C_1$-$C_{22}$ ou C-acyle en $C_1$-$C_{22}$, et $R^3$, $R^4$, $R^5$ et $R^6$ ont les significations données précédemment,

$R^{18}$ représente un atome d'hydrogène, un reste alkyle en $C_1$-$C_{22}$, qui peut être interrompu par des atomes d'oxygène d'une fonction éther, de soufre ou d'azote ou substitué par des groupements cyano, carboxyle, carbamoyle, ester carboxylique, hydroxy, thiol ou amino, ou un reste C-acyle en $C_1$-$C_{22}$,

$R^{19}$ représente un atome d'hydrogène, un reste alkyle en $C_1$-$C_{22}$, qui peut être interrompu par des atomes d'oxygène d'une fonction éther, de soufre ou d'azote ou substitué par des groupements cyano ou ester carboxylique, un reste aryle qui peut être éventuellement substitué par des groupements chloro, alkyle en $C_1$-$C_{22}$ ou alcoxy en $C_1$-$C_{22}$, un reste aralkyle en $C_7$-$C_{22}$ ou cycloalkyle en $C_3$-$C_{12}$, et

$R^{20}$ représente un atome d'hydrogène ou un reste alkyle éventuellement ramifié,

ainsi que sels d'acides d'addition et hydrates de ces composés.

2. Composés selon la revendication 1, caractérisés en ce que n est mis pour 1 à 20.

3. Composés selon la revendication 1 ou 2, caractérisés en ce que $R^1$ et $R^2$ sont choisis parmi des atomes d'hydrogène et des radicaux méthyle et phényle.

4. Composés selon l'une quelconque des revendications 1 à 3, caractérisés en ce que $R^3$, $R^4$, $R^5$ et $R^6$ sont mis pour des radicaux méthyle.

5. Composés selon l'une quelconque des revendications 1 à 4, caractérisés en ce que X et Y représentent des atomes d'oxygène.

6. Composés selon l'une quelconque des revendications 1 à 5, caractérisés en ce que -A- est mis pour un atome d'oxygène ou un atome d'azote acylé une fois.

7. Composés selon l'une quelconque des revendications 1 à 6, caractérisés en ce que -B- est une liaison directe ou un groupement alkylène.

8. Composés selon l'une quelconque des revendications 1 à 7, caractérisés en ce que -C- est mis pour un atome d'hydrogène ou un groupement C-acyle en $C_1$-$C_{20}$.

9. Mélanges de composés de formule générale I selon l'une quelconque des revendications 1 à 8.

10. Composés de formule

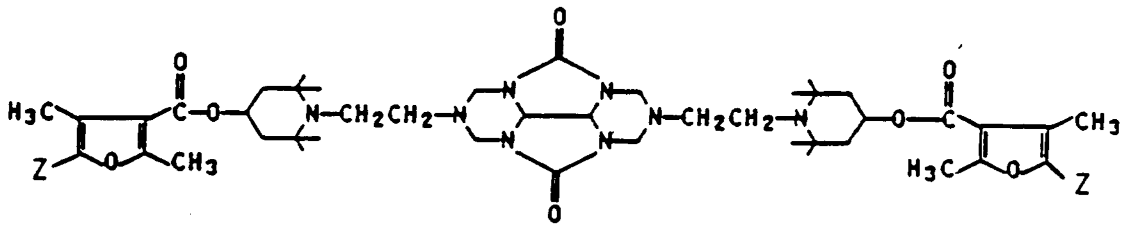

dans laquelle Z est mis pour un atome d'hydrogène ou un radical méthyle.

**11.** Utilisation des composés selon l'une quelconque des revendications 1 à 10 et de leurs mélanges pour la stabilisation de matières organiques.

**12.** Utilisation des composés selon l'une quelconque des revendications 1 à 10 et de leurs mélanges pour la stabilisation de matières plastiques.

**13.** Utilisation selon la revendication 12 pour la stabilisation de polyoléfines, de polyamides et de laques.

**14.** Utilisation selon l'une quelconque des revendications 11 à 13 pour la protection contre la lumière et la chaleur ou comme désactiveur pour métaux.

**15.** Matière organique stabilisée, contenant des composés selon l'une quelconque des revendications 1 à 10.

**16.** Utilisation selon la revendication 12 pour la stabilisation de polyuréthannes.